# EUROPEAN PATENT SPECIFICATION

(11) **EP 3 193 703 B1**
(45) Date of publication and mention of the grant of the patent: **01.07.2020**
(21) Application number: 15770777.9
(22) Date of filing: 16.09.2015
(51) Int. Cl.: A61B 5/00, A61B 5/0402

(54) **AMBULATORY ELECTROCARDIOGRAPHY MONITORING PATCH**
PFLASTER ZUR AMBULANTEN ELEKTROKARDIOGRAFIEÜBERWACHUNG
TIMBRE DE SURVEILLANCE D'ÉLECTROCARDIOGRAPHIE AMBULATOIRE

(30) Priority: 16.09.2014 US 201414488230
(43) Date of publication of application: 26.07.2017
(73) Proprietor: Bardy Diagnostics, Inc., Charlotte, NC 28207 (US)
(72) Inventor: BISHAY, Jon Mikalson, Lexington, KY 40502 (US); FELIX, Jason, Vashon Island, Washington 98070 (US); BARDY, Gust H., Carnation, Washington 98014 (US)
(74) Representative: Hanna Moore + Curley
(86) International application number: PCT/US2015/050507
(87) International publication number: WO 2016/044472

(56) References cited:
- WO-A1-2006/009767
- WO-A1-2009/036306
- WO-A2-2008/057884
- WO-A2-2010/105045
- DORTHE B SAADI ET AL: "Heart Rhythm Analysis using ECG recorded with a Novel Sternum based Patch Technology -A Pilot Study", CARDIOTECHNIX 2013 - INTERNATIONAL CONGRESS ON CARDIOVASCULAR TECHNOLOGIES, 20 September 2013 (2013-09-20), XP055157450,

## Description

### TECHNICAL FIELD

This application relates in general to electrocardiographic monitoring and, in particular, to an ambulatory electrocardiography monitoring patch optimized for capturing low amplitude cardiac action potential propagation from the atria.

### BACKGROUND ART

The first electrocardiogram (ECG) was invented by a Dutch physiologist, Willem Einthoven, in 1903, who used a string galvanometer to measure the electrical activity of the heart. Generations of physicians around the world have since used ECGs, in various forms, to diagnose heart problems and other potential medical concerns. Although the basic principles underlying Dr. Einthoven's original work, including his naming of various waveform deflections (Einthoven's triangle), are still applicable today, ECG machines have evolved from his original three-lead ECG, to ECGs with unipolar leads connected to a central reference terminal starting in 1934, to augmented unipolar leads beginning in 1942, and finally to the 12-lead ECG standardized by the American Heart Association in 1954 and still in use today. Further advances in portability and computerized interpretation have been made, yet the electronic design of the ECG recording apparatuses has remained fundamentally the same for much of the past 40 years.

Essentially, an ECG measures the electrical signals emitted by the heart as generated by the propagation of the action potentials that trigger depolarization of heart fibers. Physiologically, transmembrane ionic currents are generated within the heart during cardiac activation and recovery sequences. Cardiac depolarization originates high in the right atrium in the sinoatrial (SA) node before spreading leftward towards the left atrium and inferiorly towards the atrioventricular (AV) node. After a delay occasioned by the AV node, the depolarization impulse transits the Bundle of His and moves into the right and left bundle branches and Purkinje fibers to activate the right and left ventricles.

During each cardiac cycle, the ionic currents create an electrical field in and around the heart that can be detected by ECG electrodes placed on the skin. Cardiac electrical activity is then visually represented in an ECG trace by PQRSTU-waveforms. The P-wave represents atrial electrical activity, and the QRSTU components represent ventricular electrical activity. Specifically, a P-wave represents atrial depolarization, which causes atrial contraction.

P-wave analysis based on ECG monitoring is critical to accurate cardiac rhythm diagnosis and focuses on localizing the sites of origin and pathways of arrhythmic conditions. P-wave analysis is also used in the diagnosis of other medical disorders, including imbalance of blood chemistry. Cardiac arrhythmias are defined by the morphology of P-waves and their relationship to QRS intervals. For instance, atrial fibrillation (AF), an abnormally rapid heart rhythm, can be confirmed by an absence of P-waves and an irregular ventricular rate. Similarly, sinoatrial block is characterized by a delay in the onset of P-waves, while junctional rhythm, an abnormal heart rhythm resulting from impulses coming from a locus of tissue in the area of the AV node, usually presents without P-waves or with inverted P-waves. Also, the amplitudes of P-waves are valuable for diagnosis. The presence of broad, notched P-waves can indicate left atrial enlargement. Conversely, the presence of tall, peaked P-waves can indicate right atrial enlargement. Finally, P-waves with increased amplitude can indicate hypokalemia, caused by low blood potassium, whereas P-waves with decreased amplitude can indicate hyperkalemia, caused by elevated blood potassium.

Cardiac rhythm disorders may present with lightheadedness, fainting, chest pain, hypoxia, syncope, palpitations, and congestive heart failure (CHF), yet rhythm disorders are often sporadic in occurrence and may not show up in-clinic during a conventional 12-second ECG. Continuous ECG monitoring with P-wave-centric action potential acquisition over an extended period is more apt to capture sporadic cardiac events. However, recording sufficient ECG and related physiological data over an extended period remains a significant challenge, despite an over 40-year history of ambulatory ECG monitoring efforts combined with no appreciable improvement in P-wave acquisition techniques since Dr. Einthoven's original pioneering work over a 110 years ago.

Electrocardiographic monitoring over an extended period provides a physician with the kinds of data essential to identifying the underlying cause of sporadic cardiac conditions, especially rhythm disorders, and other physiological events of potential concern. A 30-day observation period is considered the "gold standard" of monitoring, yet a 14-day observation period is currently pitched as being achievable by conventional ECG monitoring approaches. Realizing a 30-day observation period has proven unworkable with existing ECG monitoring systems, which are arduous to employ; cumbersome, uncomfortable and not user-friendly to the patient; and costly to manufacture and deploy. Still, if a patient's ECG could be recorded in an ambulatory setting over a prolonged time periods, particularly for more than 14 days, thereby allowing the patient to engage in activities of daily living, the chances of acquiring meaningful medical information and capturing an abnormal event while the patient is engaged in normal activities are greatly improved.

The location of the atria and their low amplitude, low frequency content electrical signals make P-waves difficult to sense, particularly through ambulatory ECG monitoring. The atria are located posteriorly within the chest, and their physical distance from the skin surface adversely affects current strength and signal fidelity. Cardiac electrical potentials measured dermally have an amplitude of only one-percent of the amplitude of transmembrane electrical potentials. The distance between the heart and ECG electrodes reduces the magnitude of electrical potentials in proportion to the square of change in distance, which compounds the problem of sensing low amplitude P-waves. Moreover, the tissues and structures that lie between the activation regions within the heart and the body's surface alter the cardiac electrical field due to changes in the electrical resistivity of adjacent tissues. Thus, surface electrical potentials, when even capable of being accurately detected, are smoothed over in aspect and bear only a general spatial relationship to actual underlying cardiac events, thereby complicating diagnosis. Conventional 12-lead ECGs attempt to compensate for weak P-wave signals by monitoring the heart from multiple perspectives and angles, while conventional ambulatory ECGs primarily focus on monitoring higher amplitude ventricular activity that can be readily sensed. Both approaches are unsatisfactory with respect to the P-wave and the accurate, medically actionable diagnosis of the myriad cardiac rhythm disorders that exist.

Additionally, maintaining continual contact between ECG electrodes and the skin after a day or two of ambulatory ECG monitoring has been a problem. Time, dirt, moisture, and other environmental contaminants, as well as perspiration, skin oil, and dead skin cells from the patient's body, can get between an ECG electrode's non-conductive adhesive and the skin's surface. These factors adversely affect electrode adhesion and the quality of cardiac signal recordings. Furthermore, the physical movements of the patient and their clothing impart various compressional, tensile, bending, and torsional forces on the contact point of an ECG electrode, especially over long recording times, and an inflexibly fastened ECG electrode will be prone to becoming dislodged. Moreover, dislodgment may occur unbeknownst to the patient, making the ECG recordings worthless. Further, some patients may have skin that is susceptible to itching or irritation, and the wearing of ECG electrodes can aggravate such skin conditions. Thus, a patient may want or need to periodically remove or replace ECG electrodes during a long-term ECG monitoring period, whether to replace a dislodged electrode, reestablish better adhesion, alleviate itching or irritation, allow for cleansing of the skin, allow for showering and exercise, or for other purpose. Such replacement or slight alteration in electrode location actually facilitates the goal of recording the ECG signal for long periods of time.

Conventionally, multi-week or multi-month monitoring can be performed by implantable ECG monitors, such as the Reveal LINQ insertable cardiac monitor, manufactured by Medtronic, Inc., Minneapolis, MN. This monitor can detect and record paroxysmal or asymptomatic arrhythmias for up to three years. However, like all forms of implantable medical device (IMD), use of this monitor requires invasive surgical implantation, which significantly increases costs; requires ongoing follow up by a physician throughout the period of implantation; requires specialized equipment to retrieve monitoring data; and carries complications attendant to all surgery, including risks of infection, injury or death.

Holter monitors are widely used for extended ECG monitoring. Typically, they are often used for only 24-48 hours. A typical Holter monitor is a wearable and portable version of an ECG that include cables for each electrode placed on the skin and a separate battery-powered ECG recorder. The leads are placed in the anterior thoracic region in a manner similar to what is done with an in-clinic standard ECG machine using electrode locations that are not specifically intended for optimal P-wave capture. The duration of monitoring depends on the sensing and storage capabilities of the monitor. A "looping" Holter (or event) monitor can operate for a longer period of time by overwriting older ECG tracings, thence "recycling" storage in favor of extended operation, yet at the risk of losing event data. Although capable of extended ECG monitoring, Holter monitors are cumbersome, expensive and typically only available by medical prescription, which limits their usability. Further, the skill required to properly place the electrodes on the patient's chest precludes a patient from replacing or removing the sensing leads and usually involves moving the patient from the physician office to a specialized center within the hospital or clinic.

U.S. Patent No. 8,460,189, to Libbus et al. ("Libbus") discloses an adherent wearable cardiac monitor that includes at least two measurement electrodes and an accelerometer. The device includes a reusable electronics module and a disposable adherent patch that includes the electrodes. ECG monitoring can be conducted using multiple disposable patches adhered to different locations on the patient's body. The device includes a processor configured to control collection and transmission of data from ECG circuitry, including generating and processing of ECG signals and data acquired from two or more electrodes. The ECG circuitry can be coupled to the electrodes in many ways to define an ECG vector, and the orientation of the ECG vector can be determined in response to the polarity of the measurement electrodes and orientation of the electrode measurement axis. The accelerometer can be used to determine the orientation of
the measurement electrodes in each of the locations. The ECG signals measured at different locations can be rotated based on the accelerometer data to modify amplitude and direction of the ECG features to approximate a standard ECG vector. The signals recorded at different locations can be combined by summing a scaled version of each signal. Libbus further discloses that inner ECG electrodes may be positioned near outer electrodes to increase the voltage of measured ECG signals. However, Libbus treats ECG signal acquisition as the measurement of a simple aggregate directional data signal without differentiating between the distinct kinds of cardiac electrical activities presented with an ECG waveform, particularly atrial (P-wave) activity.

The ZIO XT Patch and ZIO Event Card devices, manufactured by iRhythm Tech., Inc., San Francisco, CA, are wearable monitoring devices that are typically worn on the upper left pectoral region to respectively provide continuous and looping ECG recording. The location is used to simulate surgically implanted monitors, but without specifically enhancing P-wave capture. Both of these devices are prescription-only and for single patient use. The ZIO XT Patch device is limited to a 14-day period, while the electrodes only of the ZIO Event Card device can be worn for up to 30 days. The ZIO XT Patch device combines both electronic recordation components and physical electrodes into a unitary assembly that adheres to the patient's skin. The ZIO XT Patch device uses adhesive sufficiently strong to support the weight of both the monitor and the electrodes over an extended period and to resist disadherence from the patient's body, albeit at the cost of disallowing removal or relocation during the monitoring period. The ZIO Event Card device is a form of downsized Holter monitor with a recorder component that must be removed temporarily during baths or other activities that could damage the non-waterproof electronics. Both devices represent compromises between length of wear and quality of ECG monitoring, especially with respect to ease of long term use, female-friendly fit, and quality of cardiac electrical potential signals, especially atrial (P-wave) signals.

WO2006/009767-A1 discloses a method and apparatus for capturing biopotential voltage signals such as electroencephalograms (EEG's), electrocardiograms (ECG's), or electromyograms (EMG's). In particular, WO 2006/009767 describes the apparatus that includes a sealed electronic module that encloses a flexible printed circuit with various integrated circuit devices such as amplifiers, analog-to-digital converters, a microcontroller, random access memory, a digital radio, a battery, and an antenna. The apparatus further includes a flexible electrode strip with at least one electrode contact affixed to each end, with a memory chip containing a digital identifier affixed to the electrode strip. Contact plugs are affixed to the electrode strip and are electrically connected to electrode pads and to the identifier memory chip. The electrode strip has an adhesive backing that allows the strip to be affixed to the patient's skin and the electrode contacts may be impregnated with an electrolyte to enhance skin conductance. The electronics module can be attached to the electrode strip by inserting the electrode strip contact plugs into mating sockets on the electronics module, which energizes the combined apparatus using the battery in the electronics module. Upon the combining, with the electronics module reading the identification data from the contact strip and configuring for appropriate gain, data capture rate, and wireless data transmission. The voltage differentials sensed are detected, amplified, and digitized in the electronics module.

"Heart Rhythm Analysis using ECG recorded with a Novel Sternum based Patch Technology - A Pilot Study" by Saadi et al., Cardoptechnix 2013- International Congress on Cardiovascular Technologies, 20 September 2013, discloses a an ePatch heart monitoring platform. The ePatch platform includes two parts: a bio-compatible, single use adhesive electrode with multiple skin contact paints that is attached to the surface of the skin ("ePatch electrode") and a reusable device that contains a rechargeable battery, electronic parts, signal processing module, data processing module, and wireless data transmission equipment ("ePatch sensor"). The sensor is attached directly to the electrode and is used to measure via two ECG channels bipolar derivations from multiple skin points. The platform was used to monitor 25 different hospitalized patients for approximately 24 hours and the monitoring results were evaluated for being diagnostically meaningful to medical doctors.

WO 2009/036306 discloses an adherent device that includes an adhesive patch that attaches to the patient's thorax and that includes at least two electrodes and an accelerometer, which is used to determine an orientation of the of the electrodes on the patient and correct measurements made by the patch. The device includes further components to transmit data collected by the device to a remote center, such as via wireless communication with an intermediate device, and can further provide wireless alerts when necessary. The device further includes electronic components to take physiological measurement, including the impedance circuitry, electrocardiogram circuitry, and a temperature sensor.

WO2008/057884 A2, teaches a body worn patient monitoring device that include at least one disposable electrode module that includes a plurality of electrical connections to the body, such as electrodes for ECG monitoring. The electrodes can be two electrodes that generate an ECG difference potential for the patient and a third, reference, electrode that can be electrically coupled to the electronic common. The reference electrode is used to ensure that inputs from the other electrodes remain within a reasonable common mode range. In a further embodiment, the electrode can be actively driven by the communications and computations module. The driving of the reference electrode is accomplished by buffering high impedance from the ECG electrodes, inverting the averaged common mode noise, and applying the inverted signal out of phase to the reference electrode. The flexible printed circuit of the disposable monitor can receive contacts such as battery clips to connect to batteries. The disposable monitor and the communications and computation module can establish an electrical connection by coupling conductive sockets of the module with an electrical plug having a plurality of conductive pins that correspond to an electrical connection pad on the flexible circuit of the communications module. The connection pads further couple the signals from the electrodes to the electrical plug via resistive traces that should survive multiple defibrillation cycles such that the monitoring device remains functional even after multiple attempts to restart the patient's heart. The traces can be used to provide a direct-connected reference electrode.

International Publication No. WO2010/105045 A2 to Corventis, Inc. discloses a device for fall protection. The device is adhered to a patient at many locations, such as the thorax of the patient along a horizontal axis of the patient, and can include a reusable electronics module and a plurality of replaceable patches, with each patch including with at least four electrodes to measure the electrocardiogram of the patient. Gel is positioned over each electrode to provide electrical conductivity between the electrodes and the skin of the patient. A printed circuit board is positioned over a gel cover placed on a surface of the patch opposite the patient's skin on which the electrodes are facing and includes an electronics layer that is encapsulated in a housing. Further, the printed circuit board is connected to the electrodes with connectors. Specifically, the connectors can include a flexible polyester film coated with conductive silver ink or insulated wires or film with conductive ink, and can be positioned on the printed circuit board in alignment with the electrodes. Each of the replaceable patches can further include a battery.

Therefore, a need remains for a low cost extended wear continuously recording ECG monitor attuned to capturing low amplitude cardiac action potential propagation for arrhythmia diagnosis, particularly atrial activation P-waves, and practicably capable of being worn for a long period of time, especially in patient's whose breast anatomy or size can interfere with signal quality in both women and men.

### DISCLOSURE OF THE INVENTION

The present application provides an ambulatory electrocardiography monitor in accordance with the claims which follow.

### DESCRIPTION OF THE DRAWINGS

FIGURES 1 and 2 are diagrams showing, by way of examples, an extended wear electrocardiography monitor, including an extended wear electrode patch, in accordance with one embodiment, respectively fitted to the sternal region of a female patient and a male patient.
FIGURE 3 is a front anatomical view showing, by way of illustration, the locations of the heart and lungs within the rib cage of an adult human.
FIGURE 4 is a perspective view showing an extended wear electrode patch in accordance with one embodiment with a monitor recorder inserted.
FIGURE 5 is a perspective view showing the monitor recorder of FIGURE 4.
FIGURE 6 is a perspective view showing the extended wear electrode patch of FIGURE 4 without a monitor recorder inserted.
FIGURE 7 is a bottom plan view of the monitor recorder of FIGURE 4.
FIGURE 8 is a top view showing the flexible circuit of the extended wear electrode patch of FIGURE 4.
FIGURE 9 is a functional block diagram showing the component architecture of the circuitry of the monitor recorder of FIGURE 4.
FIGURE 10 is a functional block diagram showing the circuitry of the extended wear electrode patch of FIGURE 4.
FIGURE 11 is a schematic diagram showing the ECG front end circuit of the circuitry of the monitor recorder of FIGURE 9.
FIGURE 12 is a flow diagram showing a monitor recorder-implemented method for monitoring ECG data for use in the monitor recorder of FIGURE 4.
FIGURE 13 is a graph showing, by way of example, a typical ECG waveform.
FIGURE 14 is a functional block diagram showing the signal processing functionality of the microcontroller.
FIGURE 15 is a functional block diagram showing the operations performed by the download station.
FIGURES 16A-C are functional block diagrams respectively showing practical uses of the extended wear electrocardiography monitors of FIGURES 1 and 2.
FIGURE 17 is a perspective view of an extended wear electrode patch with a flexile wire electrode assembly in accordance with a still further embodiment.
FIGURE 18 is perspective view of the flexile wire electrode assembly from FIGURE 17, with a layer of insulating material shielding a bare distal wire around the midsection of the flexible backing.
FIGURE 19 is a bottom view of the flexile wire electrode assembly as shown in FIGURE 17.
FIGURE 20 is a bottom view of a flexile wire electrode assembly in accordance with a still yet further embodiment.
FIGURE 21 is a perspective view showing the longitudinal midsection of the flexible backing of the electrode assembly from FIGURE 17.

### BEST MODE FOR CARRYING OUT THE INVENTION

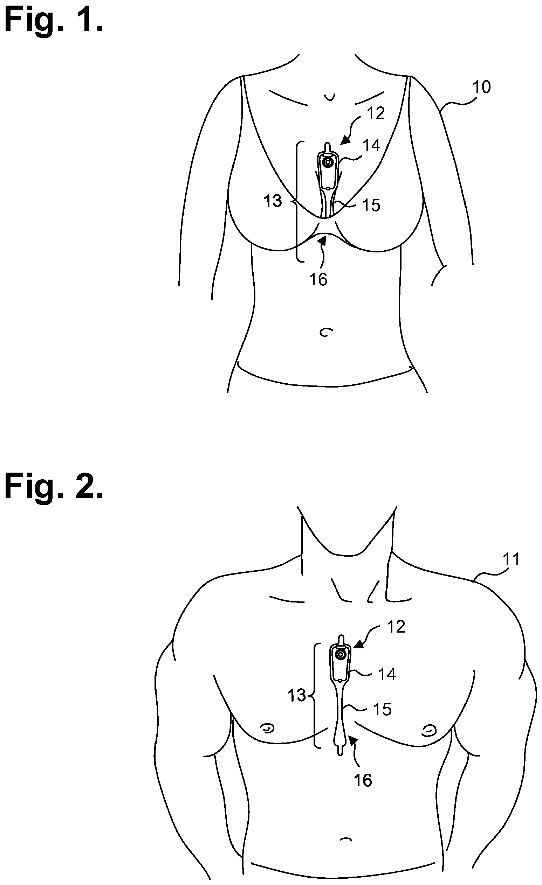

ECG and physiological monitoring can be provided through a wearable ambulatory monitor that includes two components, a flexible extended wear electrode patch and a removable reusable (or single use) monitor recorder. Both the electrode patch and the monitor recorder are optimized to capture electrical signals from the propagation of low amplitude, relatively low frequency content cardiac action potentials, particularly the P-waves generated during atrial activation. FIGURES 1 and 2 are diagrams showing, by way of examples, an extended wear electrocardiography monitor 12, including a monitor recorder 14, in accordance with one embodiment, respectively fitted to the sternal region of a female patient 10 and a male patient 11. The wearable monitor 12 sits centrally, positioned axially along the sternal midline 16, on the patient's chest along the sternum 13 and oriented top-to-bottom with the monitor recorder 14 preferably situated towards the patient's head. In a further embodiment, the orientation of the wearable monitor 12 can be corrected post-monitoring, as further described *infra*, for instance, if the wearable monitor 12 is inadvertently fitted upside down.

The electrode patch 15 is shaped to fit comfortably and conformal to the contours of the patient's chest approximately centered on the sternal midline 16 (or immediately to either side of the sternum 13). The distal end of the electrode patch 15, under which a lower or inferior pole (ECG electrode) is adhered, extends towards the Xiphoid process and lower sternum and, depending upon the patient's build, may straddle the region over the Xiphoid process and lower sternum. The proximal end of the electrode patch 15, located under the monitor recorder 14, under which an upper or superior pole (ECG electrode) is adhered, is below the manubrium and, depending upon patient's build, may straddle the region over the manubrium.

During ECG monitoring, the amplitude and strength of action potentials sensed on the body's surface are affected to varying degrees by cardiac, cellular, extracellular, vector of current flow, and physical factors, like obesity, dermatitis, large breasts, and high impedance skin, as can occur in dark-skinned individuals. Sensing along the sternal midline 16 (or immediately to either side of the sternum 13) significantly improves the ability of the wearable monitor 12 to cutaneously sense cardiac electric signals, particularly the P-wave (or atrial activity) and, to a lesser extent, the QRS interval signals in the ECG waveforms that indicate ventricular activity by countering some of the effects of these factors.

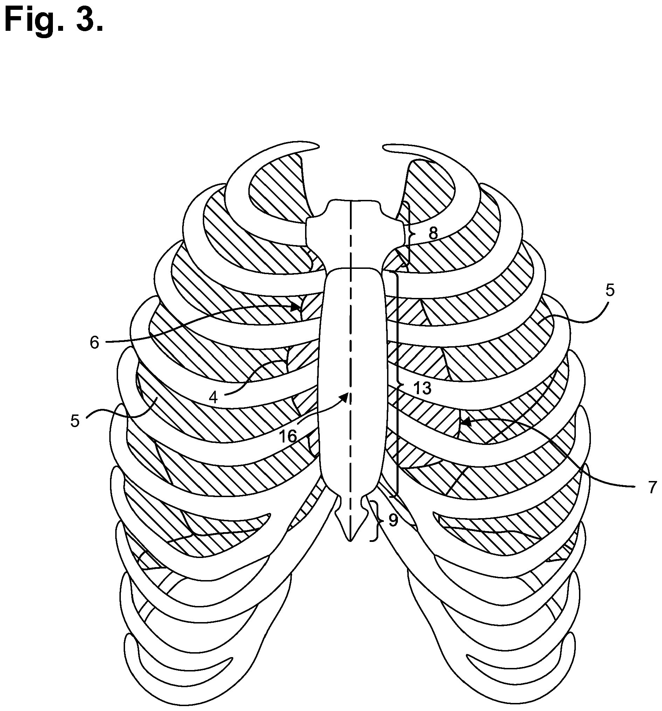

The ability to sense low amplitude, low frequency content body surface potentials is directly related to the location of ECG electrodes on the skin's surface and the ability of the sensing circuitry to capture these electrical signals. FIGURE 3 is a front anatomical view showing, by way of illustration, the locations of the heart 4 and lungs 5 within the rib cage of an adult human. Depending upon their placement locations on the chest, ECG electrodes may be separated from activation regions within the heart 4 by differing combinations of internal tissues and body structures, including heart muscle, intracardiac blood, the pericardium, intrathoracic blood and fluids, the lungs 5, skeletal muscle, bone structure, subcutaneous fat, and the skin, plus any contaminants present between the skin's surface and electrode signal pickups. The degree of amplitude degradation of cardiac transmembrane potentials increases with the number of tissue boundaries between the heart 4 and the skin's surface that are encountered. The cardiac electrical field is degraded each time the transmembrane potentials encounter a physical boundary separating adjoining tissues due to differences in the respective tissues' electrical resistances. In addition, other non-spatial factors, such as pericardial effusion, emphysema or fluid accumulation in the lungs, as further explained *infra*, can further degrade body surface potentials.

Internal tissues and body structures can adversely affect the current strength and signal fidelity of all body surface potentials, yet low amplitude cardiac action potentials, particularly the P-wave with a normative amplitude of less than 0.25 microvolts (mV) and a normative duration of less than 120 milliseconds (ms), are most apt to be negatively impacted. The atria 6 are generally located posteriorly within the thoracic cavity (with the exception of the anterior right atrium and right atrial appendage), and, physically, the left atrium constitutes the portion of the heart 4 furthest away from the surface of the skin on the chest. Conversely, the ventricles 7, which generate larger amplitude signals, generally are located anteriorly with the anterior right ventricle and most of the left ventricle situated relatively close to the skin surface on the chest, which contributes to the relatively stronger amplitudes of ventricular waveforms. Thus, the quality of P-waves (and other already-low amplitude action potential signals) is more susceptible to weakening from intervening tissues and structures than the waveforms associated with ventricular activation.

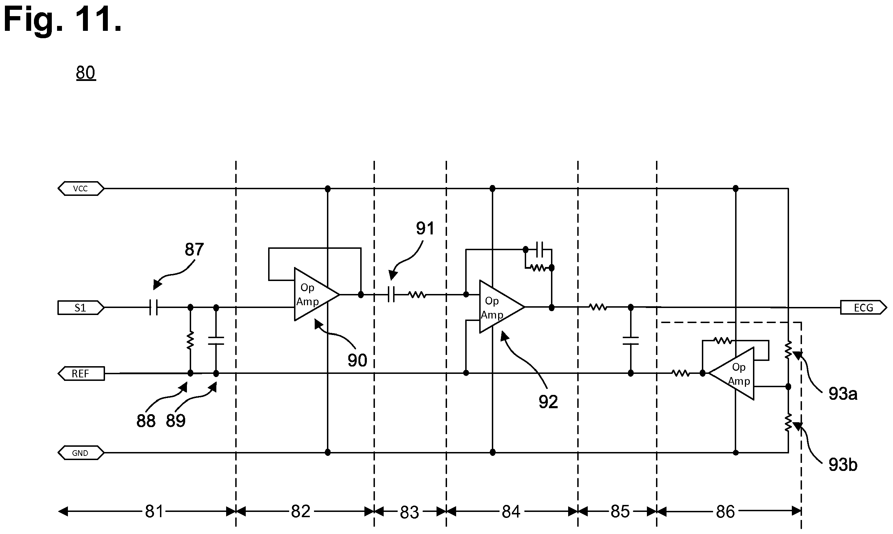

The importance of the positioning of ECG electrodes along the sternal midline 15 has largely been overlooked by conventional approaches to ECG monitoring, in part due to the inability of their sensing circuitry to reliably detect low amplitude, low frequency content electrical signals, particularly in P-waves. In turn, that inability to keenly sense P-waves has motivated ECG electrode placement in other non-sternal midline thoracic locations, where the QRSTU components that represent ventricular electrical activity are more readily detectable by their sensing circuitry than P-waves. In addition, ECG electrode placement along the sternal midline 15 presents major patient wearability challenges, such as fitting a monitoring ensemble within the narrow confines of the inter-mammary cleft between the breasts, that to large extent drive physical packaging concerns, which can be incompatible with ECG monitors intended for placement, say, in the upper pectoral region or other non-sternal midline thoracic locations. In contrast, the wearable monitor 12 uses an electrode patch 15 that is specifically intended for extended wear placement in a location at the sternal midline 16 (or immediately to either side of the sternum 13). When combined with a monitor recorder 14 that uses sensing circuitry optimized to preserve the characteristics of low amplitude cardiac action potentials, especially those signals from the atria, as further described *infra* with reference to FIGURE 11, the electrode patch 15 helps to significantly improve atrial activation (P-wave) sensing through placement in a body location that robustly minimizes the effects of tissue and body structure.

Referring back to FIGURES 1 and 2, the placement of the wearable monitor 12 in the region of the sternal midline 13 puts the ECG electrodes of the electrode patch 15 in locations better adapted to sensing and recording low amplitude cardiac action potentials during atrial propagation (P-wave signals) than placement in other locations, such as the upper left pectoral region, as commonly seen in most conventional ambulatory ECG monitors. The sternum 13 overlies the right atrium of the heart 4. As a result, action potential signals have to travel through fewer layers of tissue and structure to reach the ECG electrodes of the electrode patch 15 on the body's surface along the sternal midline 13 when compared to other monitoring locations, a distinction that is of critical importance when capturing low frequency content electrical signals, such as P-waves.

Moreover, cardiac action potential propagation travels simultaneously along a north-to-south and right-to-left vector, beginning high in the right atrium and ultimately ending in the posterior and lateral region of the left ventricle. Cardiac depolarization originates high in the right atrium in the SA node before concurrently spreading leftward towards the left atrium and inferiorly towards the AV node. The ECG electrodes of the electrode patch 15 are placed with the upper or superior pole (ECG electrode) along the sternal midline 13 in the region of the manubrium and the lower or inferior pole (ECG electrode) along the sternal midline 13 in the region of the Xiphoid process 9 and lower sternum. The ECG electrodes are placed primarily in a north-to-south orientation along the sternum 13 that corresponds to the north-to-south waveform vector exhibited during atrial activation. This orientation corresponds to the aVF lead used in a conventional 12-lead ECG that is used to sense positive or upright P-waves.

Furthermore, the thoracic region underlying the sternum 13 along the midline 16 between the manubrium 8 and Xiphoid process 9 is relatively free of lung tissue, musculature, and other internal body structures that could occlude the electrical signal path between the heart 4, particularly the atria, and ECG electrodes placed on the surface of the skin. Fewer obstructions means that cardiac electrical potentials encounter fewer boundaries between different tissues. As a result, when compared to other thoracic ECG sensing locations, the cardiac electrical field is less altered when sensed dermally along the sternal midline 13. As well, the proximity of the sternal midline 16 to the ventricles 7 facilitates sensing of right ventricular activity and provides superior recordation of the QRS interval, again, in part due to the relatively clear electrical path between the heart 4 and the skin surface.

Finally, non-spatial factors can affect transmembrane action potential shape and conductivity. For instance, myocardial ischemia, an acute cardiac condition, can cause a transient increase in blood perfusion in the lungs 5. The perfused blood can significantly increase electrical resistance across the lungs 5 and therefore degrade transmission of the cardiac electrical field to the skin's surface. However, the placement of the wearable monitor 12 along the sternal midline 16 in the inter-mammary cleft between the breasts is relatively resilient to the adverse effects to cardiac action potential degradation caused by ischemic conditions as the body surface potentials from a location relatively clear of underlying lung tissue and fat help compensate for the loss of signal amplitude and content. The monitor recorder 14 is thus able to record the P-wave morphology that may be compromised by myocardial ischemia and therefore make diagnosis of the specific arrhythmias that can be associated with myocardial ischemia more difficult.

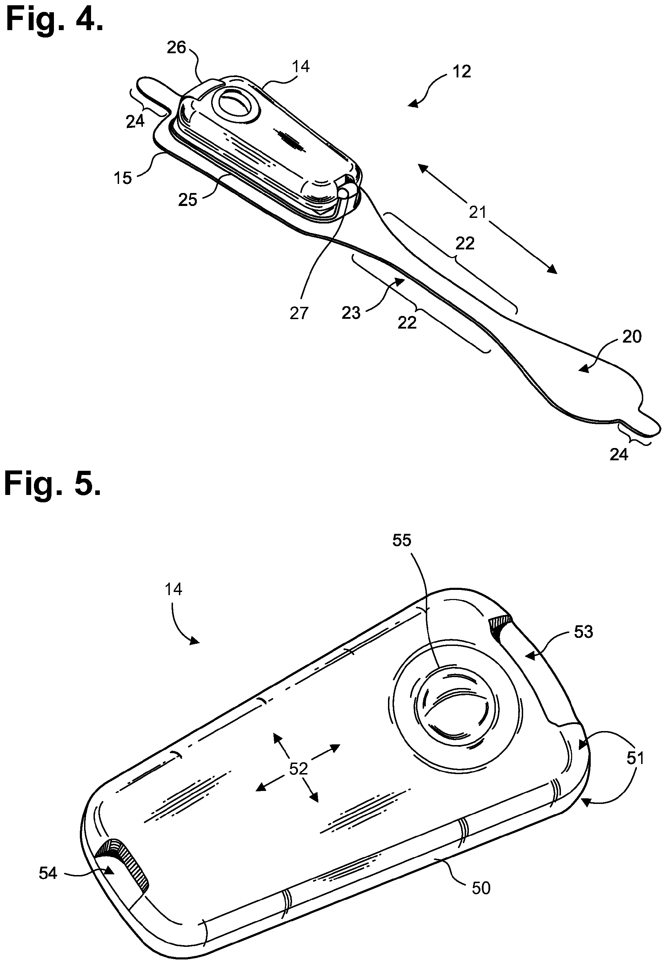

During use, the electrode patch 15 is first adhered to the skin along the sternal midline 16 (or immediately to either side of the sternum 13). A monitor recorder 14 is then snapped into place on the electrode patch 15 using an electro mechanical docking interface to initiate ECG monitoring. FIGURE 4 is a perspective view showing an extended wear electrode patch 15 in accordance with one embodiment with a monitor recorder 14 inserted. The body of the electrode patch 15 is preferably constructed using a flexible backing 20 formed as an elongated strip 21 of wrap knit or similar stretchable material about 145mm long and 32mm at the widest point with a narrow longitudinal mid-section 23 evenly tapering inward from both sides. A pair of cut-outs 22 between the distal and proximal ends of the electrode patch 15 create a narrow longitudinal midsection 23 or "isthmus" and defines an elongated "hourglass"-like shape, when viewed from above, such as described in commonly-assigned U.S. Design Patent application, entitled "Extended Wear Electrode Patch," Serial No. 29/472,045, filed November 7, 2013. The upper part of the "hourglass" is sized to allow an electrically non-conductive receptacle 25, sits on top of the outward-facing surface of the electrode patch 15, to be affixed to the electrode patch 15 with an ECG electrode placed underneath on the patient-facing underside, or contact, surface of the electrode patch 15; the upper part of the "hourglass" has a longer and wider profile (but still rounded and tapered to fit comfortably between the breasts) than the lower part of the "hourglass," which is sized primarily to allow just the placement of an ECG electrode of appropriate shape and surface area to record the P-wave and the QRS signals sufficiently given the inter-electrode spacing.

The electrode patch 15 incorporates features that significantly improve wearability, performance, and patient comfort throughout an extended monitoring period. The entire electrode patch 15 is lightweight in construction, which allows the patch to be resilient to disadhesing or falling off and, critically, to avoid creating distracting discomfort to the patient, even when the patient is asleep. In contrast, the weight of a heavy ECG monitor impedes patient mobility and will cause the monitor to constantly tug downwards and press on the patient's body that can generate skin inflammation with frequent adjustments by the patient needed to maintain comfort.

During every day wear, the electrode patch 15 is subjected to pushing, pulling, and torsional movements, including compressional and torsional forces when the patient bends forward, or tensile and torsional forces when the patient leans backwards. To counter these stress forces, the electrode patch 15 incorporates crimp and strain reliefs, such as described in commonly-assigned U.S. Patent application, entitled "Extended Wear Electrocardiography Patch," Serial No. 14/080,717, filed November 14 2013. In addition, the cut-outs 22 and longitudinal midsection 23 help minimize interference with and discomfort to breast tissue, particularly in women (and gynecomastic men). The cut-outs 22 and longitudinal midsection 23 further allow better conformity of the electrode patch 15 to sternal bowing and to the narrow isthmus of flat skin that can occur along the bottom of the inter-mammary cleft between the breasts, especially in buxom women. The cut-outs 22 and narrow and flexible longitudinal midsection 23 help the electrode patch 15 fit nicely between a pair of female breasts in the inter-mammary cleft. In one embodiment, the cut-outs 22 can be graduated to form the longitudinal midsection 23 as a narrow in-between stem or isthmus portion about 7mm wide. In a still further embodiment, tabs 24 can respectively extend an additional 8mm to 12mm beyond the distal and proximal ends of the flexible backing 20 to facilitate with adhering the electrode patch 15 to or removing the electrode patch 15 from the sternum 13. These tabs preferably lack adhesive on the underside, or contact, surface of the electrode patch 15. Still other shapes, cut-outs and conformities to the electrode patch 15 are possible.

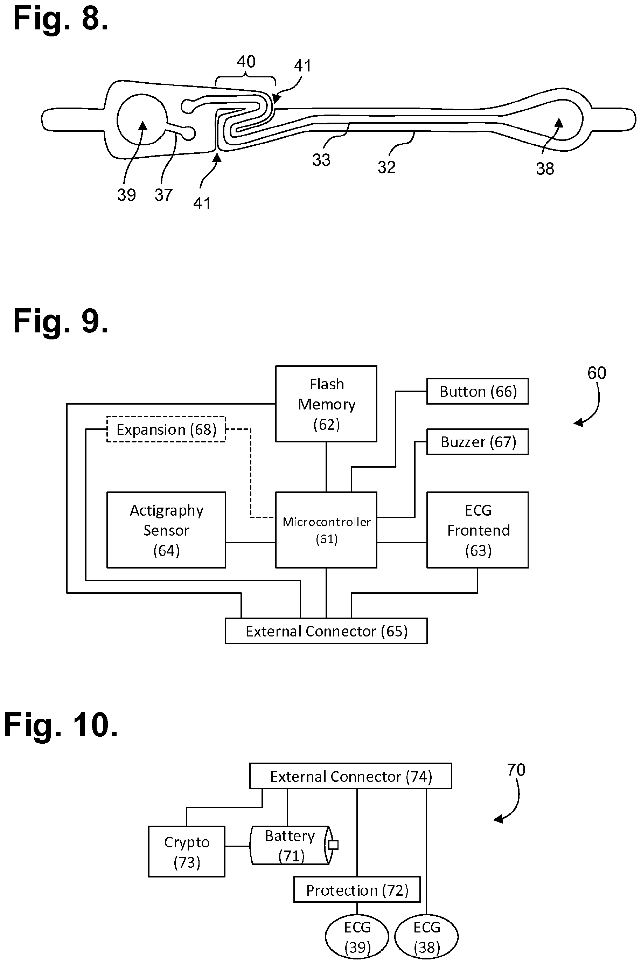

The monitor recorder 14 removably and reusably snaps into an electrically non-conductive receptacle 25 during use. The monitor recorder 14 contains electronic circuitry for recording and storing the patient's electrocardiography as sensed via a pair of ECG electrodes provided on the electrode patch 15, as further described *infra* beginning with reference to FIGURE 9. The non-conductive receptacle 25 is provided on the top surface of the flexible backing 20 with a retention catch 26 and tension clip 27 molded into the non-conductive receptacle 25 to conformably receive and securely hold the monitor recorder 14 in place.

The monitor recorder 14 includes a sealed housing that snaps into place in the non-conductive receptacle 25. FIGURE 5 is a perspective view showing the monitor recorder 14 of FIGURE 4. The sealed housing 50 of the monitor recorder 14 intentionally has a rounded isosceles trapezoidal-like shape 52, when viewed from above, such as described in commonly-assigned U.S. Design Patent application, entitled "Electrocardiography Monitor," Serial No. 29/472,046, filed November 7 2013. The edges 51 along the top and bottom surfaces are rounded for patient comfort. The sealed housing 50 is approximately 47 mm long, 23 mm wide at the widest point, and 7 mm high, excluding a patient-operable tactile-feedback button 55. The sealed housing 50 can be molded out of polycarbonate, ABS, or an alloy of those two materials. The button 55 is waterproof and the button's top outer surface is molded silicon rubber or similar soft pliable material. A retention detent 53 and tension detent 54 are molded along the edges of the top surface of the housing 50 to respectively engage the retention catch 26 and the tension clip 27 molded into non-conductive receptacle 25. Other shapes, features, and conformities of the sealed housing 50 are possible.

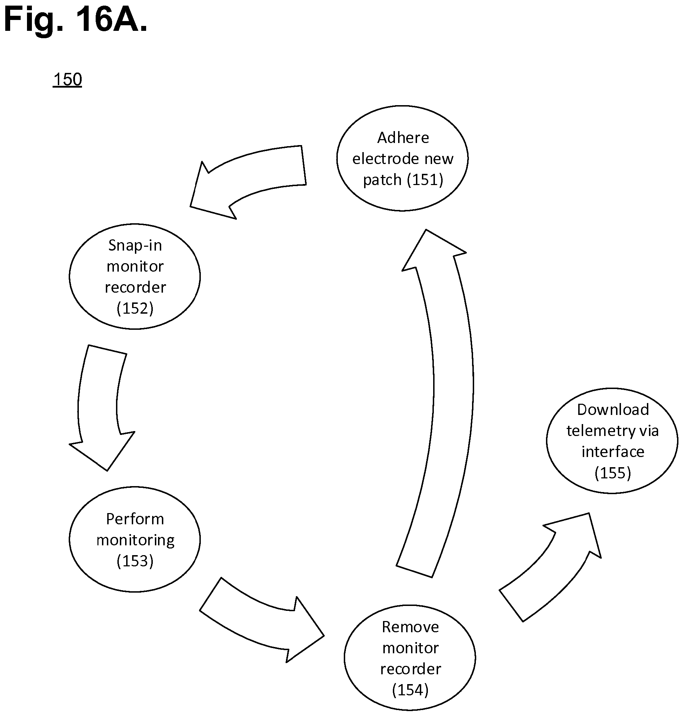

The electrode patch 15 is intended to be disposable, while the monitor recorder 14 is designed for reuse and can be transferred to successive electrode patches 15 to ensure continuity of monitoring, if so desired. The monitor recorder 14 can be used only once, but single use effectively wastes the synergistic benefits provided by the combination of the disposable electrode patch and reusable monitor recorder, as further explained *infra* with reference to FIGURES 16A-C. The placement of the wearable monitor 12 in a location at the sternal midline 16 (or immediately to either side of the sternum 13) benefits long-term extended wear by removing the requirement that ECG electrodes be continually placed in the same spots on the skin throughout the monitoring period. Instead, the patient is free to place an electrode patch 15 anywhere within the general region of the sternum 13.

As a result, at any point during ECG monitoring, the patient's skin is able to recover from the wearing of an electrode patch 15, which increases patient comfort and satisfaction, while the monitor recorder 14 ensures ECG monitoring continuity with minimal effort. A monitor recorder 14 is merely unsnapped from a worn out electrode patch 15, the worn out electrode patch 15 is removed from the skin, a new electrode patch 15 is adhered to the skin, possibly in a new spot immediately adjacent to the earlier location, and the same monitor recorder 14 is snapped into the new electrode patch 15 to reinitiate and continue the ECG monitoring.

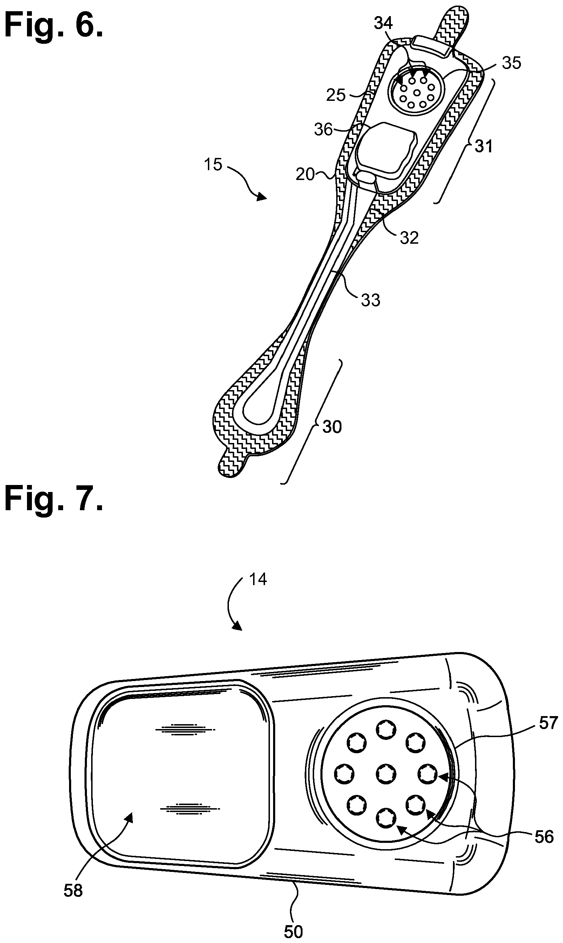

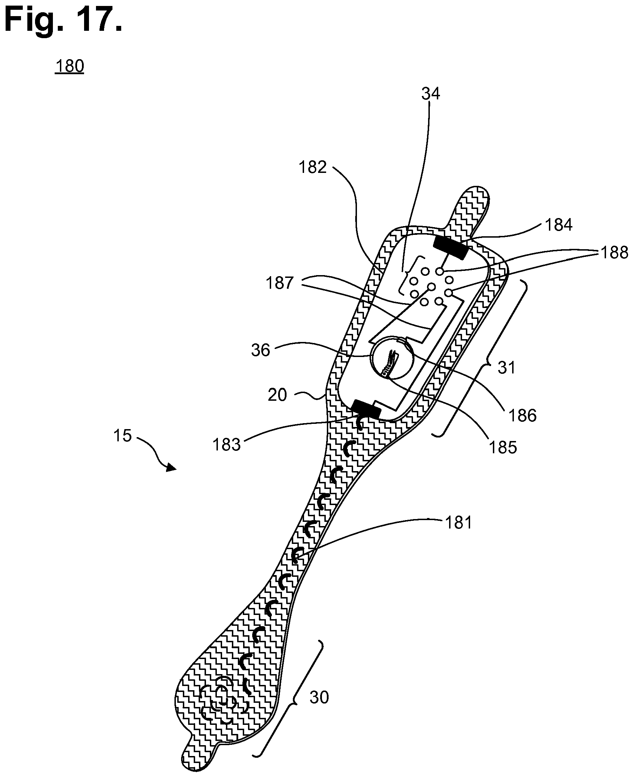

During use, the electrode patch 15 is first adhered to the skin in the sternal region. FIGURE 6 is a perspective view showing the extended wear electrode patch 15 of FIGURE 4 without a monitor recorder 14 inserted. A flexible circuit 32 is adhered to each end of the flexible backing 20. A distal circuit trace 33 from the distal end 30 of the flexible backing 20 and a proximal circuit trace (not shown) from the proximal end 31 of the flexible backing 20 electrically couple ECG electrodes (not shown) with a pair of electrical pads 34. In a further embodiment, the distal and proximal circuit traces are replaced with interlaced or sewn-in flexible wires, as further described *infra* beginning with reference to FIGURE 17. The electrical pads 34 are provided within a moisture-resistant seal 35 formed on the bottom surface of the non-conductive receptacle 25. When the monitor recorder 14 is securely received into the non-conductive receptacle 25, that is, snapped into place, the electrical pads 34 interface to electrical contacts (not shown) protruding from the bottom surface of the monitor recorder 14. The moisture-resistant seal 35 enables the monitor recorder 14 to be worn at all times, even during showering or other activities that could expose the monitor recorder 14 to moisture or adverse conditions.

In addition, a battery compartment 36 is formed on the bottom surface of the non-conductive receptacle 25. A pair of battery leads (not shown) from the battery compartment 36 to another pair of the electrical pads 34 electrically interface the battery to the monitor recorder 14. The battery contained within the battery compartment 35 is a direct current (DC) power cell and can be replaceable, rechargeable or disposable.

The monitor recorder 14 draws power externally from the battery provided in the non-conductive receptacle 25, thereby uniquely obviating the need for the monitor recorder 14 to carry a dedicated power source. FIGURE 7 is a bottom plan view of the monitor recorder 14 of FIGURE 4. A cavity 58 is formed on the bottom surface of the sealed housing 50 to accommodate the upward projection of the battery compartment 36 from the bottom surface of the non-conductive receptacle 25, when the monitor recorder 14 is secured in place on the non-conductive receptacle 25. A set of electrical contacts 56 protrude from the bottom surface of the sealed housing 50 and are arranged in alignment with the electrical pads 34 provided on the bottom surface of the non-conductive receptacle 25 to establish electrical connections between the electrode patch 15 and the monitor recorder 14. In addition, a seal coupling 57 circumferentially surrounds the set of electrical contacts 56 and securely mates with the moisture-resistant seal 35 formed on the bottom surface of the non-conductive receptacle 25. The battery contained within the battery compartment 36 can be replaceable, rechargeable or disposable. In a further embodiment, the ECG sensing circuitry of the monitor recorder 14 can be supplemented with additional sensors, including an SpO₂ sensor, a blood pressure sensor, a temperature sensor, respiratory rate sensor, a glucose sensor, an air flow sensor, and a volumetric pressure sensor, which can be incorporated directly into the monitor recorder 14 or onto the non-conductive receptacle 25.

The placement of the flexible backing 20 on the sternal midline 16 (or immediately to either side of the sternum 13) also helps to minimize the side-to-side movement of the wearable monitor 12 in the left- and right-handed directions during wear. However, the wearable monitor 12 is still susceptible to pushing, pulling, and torqueing movements, including compressional and torsional forces when the patient bends forward, and tensile and torsional forces when the patient leans backwards or twists. To counter the dislodgment of the flexible backing 20 due to compressional and torsional forces, a layer of non-irritating adhesive, such as hydrocolloid, is provided at least partially on the underside, or contact, surface of the flexible backing 20, but only on the distal end 30 and the proximal end 31. As a result, the underside, or contact surface of the longitudinal midsection 23 does not have an adhesive layer and remains free to move relative to the skin. Thus, the longitudinal midsection 23 forms a crimp relief that respectively facilitates compression and twisting of the flexible backing 20 in response to compressional and torsional forces. Other forms of flexible backing crimp reliefs are possible.

Unlike the flexible backing 20, the flexible circuit 32 is only able to bend and cannot stretch in a planar direction. The flexible circuit 32 can be provided either above or below the flexible backing 20. FIGURE 8 is a top view showing the flexible circuit 32 of the extended wear electrode patch 15 of FIGURE 4 when mounted above the flexible backing 20. A distal ECG electrode 38 and proximal ECG electrode 39 are respectively coupled to the distal and proximal ends of the flexible circuit 32 to serve as electrode signal pickups. The flexible circuit 32 preferably does not extend to the outside edges of the flexible backing 20, thereby avoiding gouging or discomforting the patient's skin during extended wear, such as when sleeping on the side. During wear, the ECG electrodes 38, 39 must remain in continual contact with the skin. A strain relief 40 is defined in the flexible circuit 32 at a location that is partially underneath the battery compartment 36 when the flexible circuit 32 is affixed to the flexible backing 20. The strain relief 40 is laterally extendable to counter dislodgment of the ECG electrodes 38, 39 due to bending, tensile and torsional forces. A pair of strain relief cutouts 41 partially extend transversely from each opposite side of the flexible circuit 32 and continue longitudinally towards each other to define in 'S'-shaped pattern, when viewed from above. The strain relief respectively facilitates longitudinal extension and twisting of the flexible circuit 32 in response to tensile and torsional forces. Other forms of circuit board strain relief are possible.

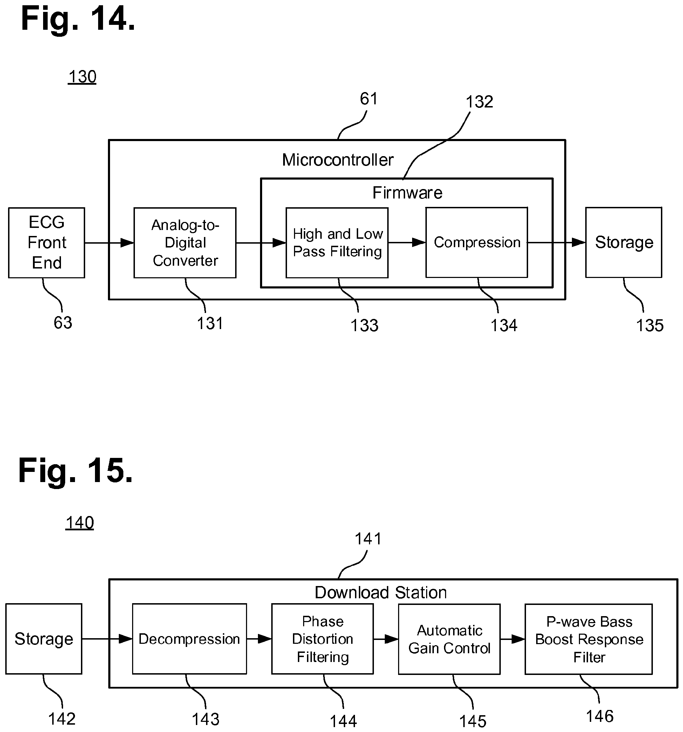

ECG monitoring and other functions performed by the monitor recorder 14 are provided through a micro controlled architecture. FIGURE 9 is a functional block diagram showing the component architecture of the circuitry 60 of the monitor recorder 14 of FIGURE 4. The circuitry 60 is externally powered through a battery provided in the non-conductive receptacle 25 (shown in FIGURE 6). Both power and raw ECG signals, which originate in the pair of ECG electrodes 38, 39 (shown in FIGURE 8) on the distal and proximal ends of the electrode patch 15, are received through an external connector 65 that mates with a corresponding physical connector on the electrode patch 15. The external connector 65 includes the set of electrical contacts 56 that protrude from the bottom surface of the sealed housing 50 and which physically and electrically interface with the set of pads 34 provided on the bottom surface of the non-conductive receptacle 25. The external connector includes electrical contacts 56 for data download, microcontroller communications, power, analog inputs, and a peripheral expansion port. The arrangement of the pins on the electrical connector 65 of the monitor recorder 14 and the device into which the monitor recorder 14 is attached, whether an electrode patch 15 or download station (not shown), follow the same electrical pin assignment convention to facilitate interoperability. The external connector 65 also serves as a physical interface to a download station that permits the retrieval of stored ECG monitoring data, communication with the monitor recorder 14, and performance of other functions. The download station is further described *infra* with reference to FIGURE 15.

Operation of the circuitry 60 of the monitor recorder 14 is managed by a microcontroller 61, such as the EFM32 Tiny Gecko 32-bit microcontroller, manufactured by Silicon Laboratories Inc., Austin, TX. The microcontroller 61 has flexible energy management modes and includes a direct memory access controller and built-in analog-to-digital and digital-to-analog converters (ADC and DAC, respectively). The microcontroller 61 also includes a program memory unit containing internal flash memory that is readable and writeable. The internal flash memory can also be programmed externally. The microcontroller 61 operates under modular micro program control as specified in firmware stored in the internal flash memory. The functionality and firmware modules relating to signal processing by the microcontroller 61 are further described *infra* with reference to FIGURE 14. The microcontroller 61 draws power externally from the battery provided on the electrode patch 15 via a pair of the electrical contacts 56. The microcontroller 61 connects to the ECG front end circuit 63 that measures raw cutaneous electrical signals using a driven reference that eliminates common mode noise, as further described *infra* with reference to FIGURE 11.

The circuitry 60 of the monitor recorder 14 also includes a flash memory 62, which the microcontroller 61 uses for storing ECG monitoring data and other physiology and information. The flash memory 62 also draws power externally from the battery provided on the electrode patch 15 via a pair of the electrical contacts 56. Data is stored in a serial flash memory circuit, which supports read, erase and program operations over a communications bus. The flash memory 62 enables the microcontroller 61 to store digitized ECG data. The communications bus further enables the flash memory 62 to be directly accessed externally over the external connector 65 when the monitor recorder 14 is interfaced to a download station.

The microcontroller 61 includes functionality that enables the acquisition of samples of analog ECG signals, which are converted into a digital representation, as further described *infra* with reference to FIGURE 14. In one mode, the microcontroller 61 will acquire, sample, digitize, signal process, and store digitized ECG data into available storage locations in the flash memory 62 until all memory storage locations are filled, after which the digitized ECG data needs to be downloaded or erased to restore memory capacity. Data download or erasure can also occur before all storage locations are filled, which would free up memory space sooner, albeit at the cost of possibly interrupting monitoring while downloading or erasure is performed. In another mode, the microcontroller 61 can include a loop recorder feature that will overwrite the oldest stored data once all storage locations are filled, albeit at the cost of potentially losing the stored data that was overwritten, if not previously downloaded. Still other modes of data storage and capacity recovery are possible.

The circuitry 60 of the monitor recorder 14 further includes an actigraphy sensor 64 implemented as a 3-axis accelerometer. The accelerometer may be configured to generate interrupt signals to the microcontroller 61 by independent initial wake up and free fall events, as well as by device position. In addition, the actigraphy provided by the accelerometer can be used during post-monitoring analysis to correct the orientation of the monitor recorder 14 if, for instance, the monitor recorder 14 has been inadvertently installed upside down, that is, with the monitor recorder 14 oriented on the electrode patch 15 towards the patient's feet, as well as for other event occurrence analyses.

The microcontroller 61 includes an expansion port that also utilizes the communications bus. External devices, separately drawing power externally from the battery provided on the electrode patch 15 or other source, can interface to the microcontroller 61 over the expansion port in half duplex mode. For instance, an external physiology sensor can be provided as part of the circuitry 60 of the monitor recorder 14, or can be provided on the electrode patch 15 with communication with the microcontroller 61 provided over one of the electrical contacts 56. The physiology sensor can include an SpO₂ sensor, blood pressure sensor, temperature sensor, respiratory rate sensor, glucose sensor, airflow sensor, volumetric pressure sensing, or other types of sensor or telemetric input sources. In a further embodiment, a wireless interface for interfacing with other wearable (or implantable) physiology monitors, as well as data offload and programming, can be provided as part of the circuitry 60 of the monitor recorder 14, or can be provided on the electrode patch 15 with communication with the microcontroller 61 provided over one of the electrical contacts 56.

Finally, the circuitry 60 of the monitor recorder 14 includes patient-interfaceable components, including a tactile feedback button 66, which a patient can press to mark events or to perform other functions, and a buzzer 67, such as a speaker, magnetic resonator or piezoelectric buzzer. The buzzer 67 can be used by the microcontroller 61 to output feedback to a patient such as to confirm power up and initiation of ECG monitoring. Still other components as part of the circuitry 60 of the monitor recorder 14 are possible.

While the monitor recorder 14 operates under micro control, most of the electrical components of the electrode patch 15 operate passively. FIGURE 10 is a functional block diagram showing the circuitry 70 of the extended wear electrode patch 15 of FIGURE 4. The circuitry 70 of the electrode patch 15 is electrically coupled with the circuitry 60 of the monitor recorder 14 through an external connector 74. The external connector 74 is terminated through the set of pads 34 provided on the bottom of the non-conductive receptacle 25, which electrically mate to corresponding electrical contacts 56 protruding from the bottom surface of the sealed housing 50 to electrically interface the monitor recorder 14 to the electrode patch 15.

The circuitry 70 of the electrode patch 15 performs three primary functions. First, a battery 71 is provided in a battery compartment formed on the bottom surface of the non-conductive receptacle 25. The battery 71 is electrically interfaced to the circuitry 60 of the monitor recorder 14 as a source of external power. The unique provisioning of the battery 71 on the electrode patch 15 provides several advantages. First, the locating of the battery 71 physically on the electrode patch 15 lowers the center of gravity of the overall wearable monitor 12 and thereby helps to minimize shear forces and the effects of movements of the patient and clothing. Moreover, the housing 50 of the monitor recorder 14 is sealed against moisture and providing power externally avoids having to either periodically open the housing 50 for the battery replacement, which also creates the potential for moisture intrusion and human error, or to recharge the battery, which can potentially take the monitor recorder 14 offline for hours at a time. In addition, the electrode patch 15 is intended to be disposable, while the monitor recorder 14 is a reusable component. Each time that the electrode patch 15 is replaced, a fresh battery is provided for the use of the monitor recorder 14, which enhances ECG monitoring performance quality and duration of use. Also, the architecture of the monitor recorder 14 is open, in that other physiology sensors or components can be added by virtue of the expansion port of the microcontroller 61. Requiring those additional sensors or components to draw power from a source external to the monitor recorder 14 keeps power considerations independent of the monitor recorder 14. This approach also enables a battery of higher capacity to be introduced when needed to support the additional sensors or components without effecting the monitor recorders circuitry 60.

Second, the pair of ECG electrodes 38, 39 respectively provided on the distal and proximal ends of the flexible circuit 32 are electrically coupled to the set of pads 34 provided on the bottom of the non-conductive receptacle 25 by way of their respective circuit traces 33, 37. The signal ECG electrode 39 includes a protection circuit 72, which is an inline resistor that protects the patient from excessive leakage current should the front end circuit fail.

Last, in a further embodiment, the circuitry 70 of the electrode patch 15 includes a cryptographic circuit 73 to authenticate an electrode patch 15 for use with a monitor recorder 14. The cryptographic circuit 73 includes a device capable of secure authentication and validation. The cryptographic device 73 ensures that only genuine, non-expired, safe, and authenticated electrode patches 15 are permitted to provide monitoring data to a monitor recorder 14 and for a specific patient.

The ECG front end circuit 63 measures raw cutaneous electrical signals using a driven reference that effectively reduces common mode noise, power supply noise and system noise, which is critical to preserving the characteristics of low amplitude cardiac action potentials, especially those signals from the atria. FIGURE 11 is a schematic diagram 80 showing the ECG front end circuit 63 of the circuitry 60 of the monitor recorder 14 of FIGURE 9. The ECG front end circuit 63 senses body surface potentials through a signal lead ("S1") and reference lead ("REF") that are respectively connected to the ECG electrodes of the electrode patch 15. Power is provided to the ECG front end circuit 63 through a pair of DC power leads ("VCC" and "GND"). An analog ECG signal ("ECG") representative of the electrical activity of the patient's heart over time is output, which the micro controller 11 converts to digital representation and filters, as further described *infra*.

The ECG front end circuit 63 is organized into five stages, a passive input filter stage 81, a unity gain voltage follower stage 82, a passive high pass filtering stage 83, a voltage amplification and active filtering stage 84, and an anti-aliasing passive filter stage 85, plus a reference generator. Each of these stages and the reference generator will now be described.

The passive input filter stage 81 includes the parasitic impedance of the ECG electrodes 38, 39 (shown in FIGURE 8), the protection resistor that is included as part of the protection circuit 72 of the ECG electrode 39 (shown in FIGURE 10), an AC coupling capacitor 87, a termination resistor 88, and filter capacitor 89. This stage passively shifts the frequency response poles downward there is a high electrode impedance from the patient on the signal lead S1 and reference lead REF, which reduces high frequency noise.

The unity gain voltage follower stage 82 provides a unity voltage gain that allows current amplification by an Operational Amplifier ("Op Amp") 90. In this stage, the voltage stays the same as the input, but more current is available to feed additional stages. This configuration allows a very high input impedance, so as not to disrupt the body surface potentials or the filtering effect of the previous stage.

The passive high pass filtering stage 83 is a high pass filter that removes baseline wander and any offset generated from the previous stage. Adding an AC coupling capacitor 91 after the Op Amp 90 allows the use of lower cost components, while increasing signal fidelity.

The voltage amplification and active filtering stage 84 amplifies the voltage of the input signal through Op Amp 91, while applying a low pass filter. The DC bias of the input signal is automatically centered in the highest performance input region of the Op Amp 91 because of the AC coupling capacitor 91.

The anti-aliasing passive filter stage 85 provides an anti-aliasing low pass filter. When the microcontroller 61 acquires a sample of the analog input signal, a disruption in the signal occurs as a sample and hold capacitor that is internal to the microcontroller 61 is charged to supply signal for acquisition.

The reference generator in subcircuit 86 drives a driven reference containing power supply noise and system noise to the reference lead REF. A coupling capacitor 87 is included on the signal lead S1 and a pair of resistors 93a, 93b inject system noise into the reference lead REF. The reference generator is connected directly to the patient, thereby avoiding the thermal noise of the protection resistor that is included as part of the protection circuit 72.

In contrast, conventional ECG lead configurations try to balance signal and reference lead connections. The conventional approach suffers from the introduction of differential thermal noise, lower input common mode rejection, increased power supply noise, increased system noise, and differential voltages between the patient reference and the reference used on the device that can obscure, at times, extremely, low amplitude body surface potentials.

Here, the parasitic impedance of the ECG electrodes 38, 39, the protection resistor that is included as part of the protection circuit 72 and the coupling capacitor 87 allow the reference lead REF to be connected directly to the skin's surface without any further components. As a result, the differential thermal noise problem caused by pairing protection resistors to signal and reference leads, as used in conventional approaches, is avoided.

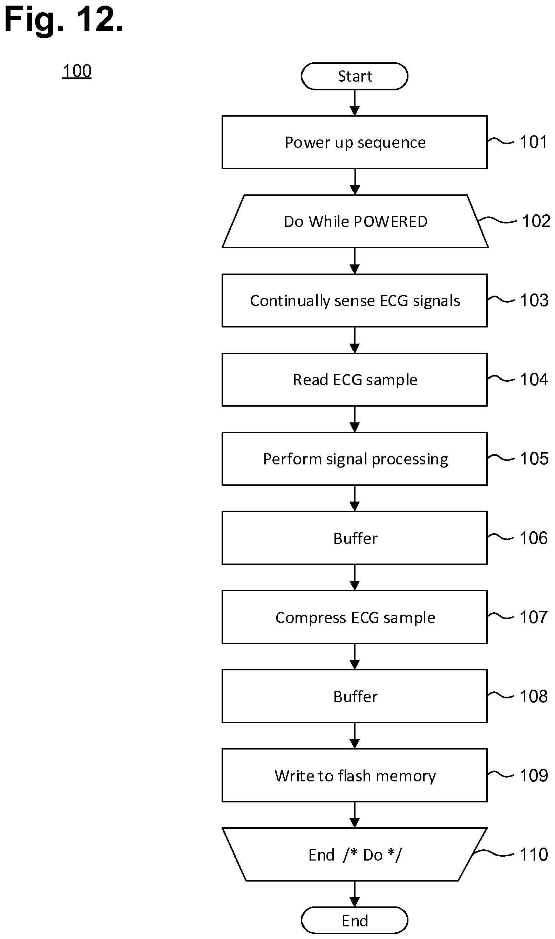

The monitor recorder 14 continuously monitors the patient's heart rate and physiology. FIGURE 12 is a flow diagram showing a monitor recorder-implemented method 100 for monitoring ECG data for use in the monitor recorder 14 of FIGURE 4. Initially, upon being connected to the set of pads 34 provided with the non-conductive receptacle 25 when the monitor recorder 14 is snapped into place, the microcontroller 61 executes a power up sequence (step 101). During the power up sequence, the voltage of the battery 71 is checked, the state of the flash memory 62 is confirmed, both in terms of operability check and available capacity, and microcontroller operation is diagnostically confirmed. In a further embodiment, an authentication procedure between the microcontroller 61 and the electrode patch 15 are also performed.

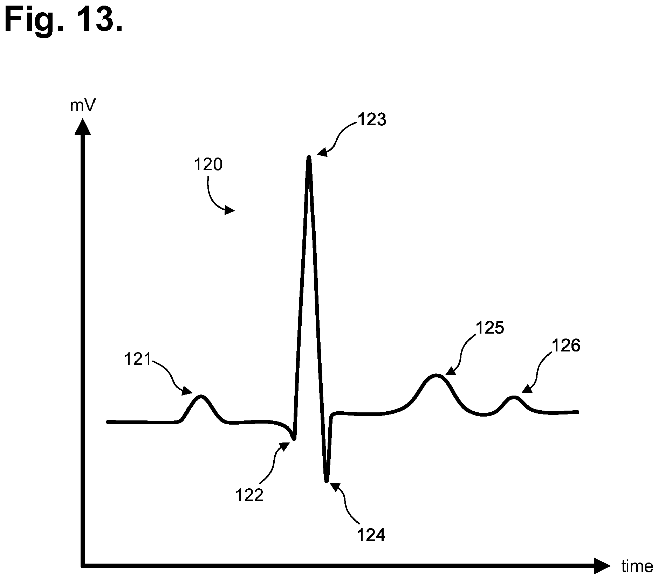

Following satisfactory completion of the power up sequence, an iterative processing loop (steps 102-110) is continually executed by the microcontroller 61. During each iteration (step 102) of the processing loop, the ECG frontend 63 (shown in FIGURE 9) continually senses the cutaneous ECG electrical signals (step 103) via the ECG electrodes 38, 29 and is optimized to maintain the integrity of the P-wave. A sample of the ECG signal is read (step 104) by the microcontroller 61 by sampling the analog ECG signal that is output by the ECG front end circuit 63. FIGURE 13 is a graph showing, by way of example, a typical ECG waveform 120. The x-axis represents time in approximate units of tenths of a second. The *y*-axis represents cutaneous electrical signal strength in approximate units of millivolts. The P-wave 121 has a smooth, normally upward, that is, positive, waveform that indicates atrial depolarization. The QRS complex often begins with the downward deflection of a Q-wave 122, followed by a larger upward deflection of an R-wave 123, and terminated with a downward waveform of the S-wave 124, collectively representative of ventricular depolarization. The T-wave 125 is normally a modest upward waveform, representative of ventricular depolarization, while the U-wave 126, often not directly observable, indicates the recovery period of the Purkinje conduction fibers.

Sampling of the R-to-R interval enables heart rate information derivation. For instance, the R-to-R interval represents the ventricular rate and rhythm, while the P-to-P interval represents the atrial rate and rhythm. Importantly, the PR interval is indicative of atrioventricular (AV) conduction time and abnormalities in the PR interval can reveal underlying heart disorders, thus representing another reason why the P-wave quality achievable by the ambulatory electrocardiography monitoring patch optimized for capturing low amplitude cardiac action potential propagation described herein is medically unique and important. The long-term observation of these ECG indicia, as provided through extended wear of the wearable monitor 12, provides valuable insights to the patient's cardiac function symptoms, and overall well-being.

Referring back to FIGURE 12, each sampled ECG signal, in quantized and digitized form, is processed by signal processing modules as specified in firmware (step 105), as described *infra*, and temporarily staged in a buffer (step 106), pending compression preparatory to storage in the flash memory 62 (step 107). Following compression, the compressed ECG digitized sample is again buffered (step 108), then written to the flash memory 62 (step 109) using the communications bus. Processing continues (step 110), so long as the monitoring recorder 14 remains connected to the electrode patch 15 (and storage space remains available in the flash memory 62), after which the processing loop is exited (step 110) and execution terminates. Still other operations and steps are possible.

The microcontroller 61 operates under modular micro program control as specified in firmware, and the program control includes processing of the analog ECG signal output by the ECG front end circuit 63. FIGURE 14 is a functional block diagram showing the signal processing functionality 130 of the microcontroller 61. The microcontroller 61 operates under modular micro program control as specified in firmware 132. The firmware modules 132 include high and low pass filtering 133, and compression 134. Other modules are possible. The microcontroller 61 has a built-in ADC, although ADC functionality could also be provided in the firmware 132.

The ECG front end circuit 63 first outputs an analog ECG signal, which the ADC 131 acquires, samples and converts into an uncompressed digital representation. The microcontroller 61 includes one or more firmware modules 133 that perform filtering. In one embodiment, three low pass filters and two high pass filters are used. Following filtering, the digital representation of the cardiac activation wave front amplitudes are compressed by a compression module 134 before being written out to storage 135.

The download station executes a communications or offload program ("Offload") or similar program that interacts with the monitor recorder 14 via the external connector 65 to retrieve the stored ECG monitoring data. FIGURE 15 is a functional block diagram showing the operations 140 performed by the download station. The download station could be a server, personal computer, tablet or handheld computer, smart mobile device, or purpose-built programmer designed specific to the task of interfacing with a monitor recorder 14. Still other forms of download station are possible, including download stations connected through wireless interfacing using, for instance, a smart phone connected to the monitor recorder 14 through Bluetooth or Wi-Fi.

The download station is responsible for offloading stored ECG monitoring data from a monitor recorder 14 and includes an electro mechanical docking interface by which the monitor recorder 14 is connected at the external connector 65. The download station operates under programmable control as specified in software 141. The stored ECG monitoring data retrieved from storage 142 on a monitor recorder 14 is first decompressed by a decompression module 143, which converts the stored ECG monitoring data back into an uncompressed digital representation more suited to signal processing than a compressed signal. The retrieved ECG monitoring data may be stored into local storage for archival purposes, either in original compressed form, or as uncompressed.

The download station can include an array of filtering modules. For instance, a set of phase distortion filtering tools 144 may be provided, where corresponding software filters can be provided for each filter implemented in the firmware executed by the microcontroller 61. The digital signals are run through the software filters in a reverse direction to remove phase distortion. For instance, a 45 Hertz high pass filter in firmware may have a matching reverse 45 Hertz high pass filter in software. Most of the phase distortion is corrected, that is, canceled to eliminate noise at the set frequency, but data at other frequencies in the waveform remain unaltered. As well, bidirectional impulse infinite response (IIR) high pass filters and reverse direction (symmetric) IIR low pass filters can be provided. Data is run through these filters first in a forward direction, then in a reverse direction, which generates a square of the response and cancels out any phase distortion. This type of signal processing is particularly helpful with improving the display of the ST-segment by removing low frequency noise.

An automatic gain control (AGC) module 145 can also be provided to adjust the digital signals to a usable level based on peak or average signal level or other metric. AGC is particularly critical to single-lead ECG monitors, where physical factors, such as the tilt of the heart, can affect the electrical field generated. On three-lead Holter monitors, the leads are oriented in vertical, horizontal and diagonal directions. As a result, the horizontal and diagonal leads may be higher amplitude and ECG interpretation will be based on one or both of the higher amplitude leads. In contrast, the electrocardiography monitor 12 has only a single lead that is oriented in the vertical direction, so variations in amplitude will be wider than available with multi-lead monitors, which have alternate leads to fall back upon.

In addition, AGC may be necessary to maintain compatibility with existing ECG interpretation software, which is typically calibrated for multi-lead ECG monitors for viewing signals over a narrow range of amplitudes. Through the AGC module 145, the gain of signals recorded by the monitor recorder 14 of the electrocardiography monitor 12 can be attenuated up (or down) to work with FDA-approved commercially available ECG interpretation.

AGC can be implemented in a fixed fashion that is uniformly applied to all signals in an ECG recording, adjusted as appropriate on a recording-by-recording basis. Typically, a fixed AGC value is calculated based on how an ECG recording is received to preserve the amplitude relationship between the signals. Alternatively, AGC can be varied dynamically throughout an ECG recording, where signals in different segments of an ECG recording are amplified up (or down) by differing amounts of gain.

Typically, the monitor recorder 14 will record a high resolution, low frequency signal for the P-wave segment. However, for some patients, the result may still be a visually small signal. Although high resolution is present, the unaided eye will normally be unable to discern the P-wave segment. Therefore, gaining the signal is critical to visually depicting P-wave detail. This technique works most efficaciously with a raw signal with low noise and high resolution, as generated by the monitor recorder 14. Automatic gain control applied to a high noise signal will only exacerbate noise content and be self-defeating.

Finally, the download station can include filtering modules specifically intended to enhance P-wave content. For instance, a P-wave base boost filter 146, which is a form of pre-emphasis filter, can be applied to the signal to restore missing frequency content or to correct phase distortion. Still other filters and types of signal processing are possible.

Conventional ECG monitors, like Holter monitors, invariably require specialized training on proper placement of leads and on the operation of recording apparatuses, plus support equipment purpose-built to retrieve, convert, and store ECG monitoring data. In contrast, the electrocardiography monitor 12 simplifies monitoring from end to end, starting with placement, then with use, and finally with data retrieval. FIGURES 16A-C are functional block diagrams respectively showing practical uses 150, 160, 170 of the extended wear electrocardiography monitors 12 of FIGURES 1 and 2. The combination of a flexible extended wear electrode patch and a removable reusable (or single use) monitor recorder empowers physicians and patients alike with the ability to readily perform long-term ambulatory monitoring of the ECG and physiology.

Especially when compared to existing Holter-type monitors and monitoring patches placed in the upper pectoral region, the electrocardiography monitor 12 offers superior patient comfort, convenience and user-friendliness. To start, the electrode patch 15 is specifically designed for ease of use by a patient (or caregiver); assistance by professional medical personnel is not required. Moreover, the patient is free to replace the electrode patch 15 at any time and need not wait for a doctor's appointment to have a new electrode patch 15 placed. In addition, the monitor recorder 14 operates automatically and the patient only need snap the monitor recorder 14 into place on the electrode patch 15 to initiate ECG monitoring. Thus, the synergistic combination of the electrode patch 15 and monitor recorder 14 makes the use of the electrocardiography monitor 12 a reliable and virtually foolproof way to monitor a patient's ECG and physiology for an extended, or even open-ended, period of time.

In simplest form, extended wear monitoring can be performed by using the same monitor recorder 14 inserted into a succession of fresh new electrode patches 15. As needed, the electrode patch 15 can be replaced by the patient (or caregiver) with a fresh new electrode patch 15 throughout the overall monitoring period. Referring first to FIGURE 16A, at the outset of monitoring, a patient adheres a new electrode patch 15 in a location at the sternal midline 16 (or immediately to either side of the sternum 13) oriented top-to-bottom (step 151). The placement of the wearable monitor in a location at the sternal midline (or immediately to either side of the sternum), with its unique narrow "hourglass"-like shape, significantly improves the ability of the wearable monitor to cutaneously sense cardiac electrical potential signals, particularly the P-wave (or atrial activity) and, to a lesser extent, the QRS interval signals indicating ventricular activity in the ECG waveforms.

Placement involves simply adhering the electrode patch 15 on the skin along the sternal midline 16 (or immediately to either side of the sternum 13). Patients can easily be taught to find the physical landmarks on the body necessary for proper placement of the electrode patch 15. The physical landmarks are locations on the surface of the body that are already familiar to patients, including the inter-mammary cleft between the breasts above the manubrium (particularly easily locatable by women and gynecomastic men), the sternal notch immediately above the manubrium, and the Xiphoid process located at the bottom of the sternum. Empowering patients with the knowledge to place the electrode patch 15 in the right place ensures that the ECG electrodes will be correctly positioned on the skin, no matter the number of times that the electrode patch 15 is replaced.

A monitor recorder 14 is snapped into the non-conductive receptacle 25 on the outward-facing surface of the electrode patch 15 (step 152). The monitor recorder 14 draws power externally from a battery provided in the non-conductive receptacle 25. In addition, the battery is replaced each time that a fresh new electrode patch 15 is placed on the skin, which ensures that the monitor recorder 14 is always operating with a fresh power supply and minimizing the chances of a loss of monitoring continuity due to a depleted battery source.

By default, the monitor recorder 14 automatically initiates monitoring upon sensing body surface potentials through the pair of ECG electrodes (step 153). In a further embodiment, the monitor recorder 14 can be configured for manual operation, such as by using the tactile feedback button 66 on the outside of the sealed housing 50, or other user-operable control. In an even further embodiment, the monitor recorder 14 can be configured for remotely-controlled operation by equipping the monitor recorder 14 with a wireless transceiver, such as described in commonly-assigned U.S. Patent application, entitled "Remote Interfacing of an Extended Wear Electrocardiography and Physiological Sensor Monitor," Serial No. 14/082,071, filed November 15, 2013. The wireless transceiver allows wearable or mobile communications devices to wirelessly interface with the monitor recorder 14.

A key feature of the extended wear electrocardiography monitor 12 is the ability to monitor ECG and physiological data for an extended period of time, which can be well in excess of the 14 days currently pitched as being achievable by conventional ECG monitoring approaches. In a further embodiment, ECG monitoring can even be performed over an open-ended time period, as further explained *infra*. The monitor recorder 14 is reusable and, if so desired, can be transferred to successive electrode patches 15 to ensure continuity of monitoring. At any point during ECG monitoring, a patient (or caregiver) can remove the monitor recorder 14 (step 154) and replace the electrode patch 15 currently being worn with a fresh new electrode patch 15 (step 151). The electrode patch 15 may need to be replaced for any number of reasons. For instance, the electrode patch 15 may be starting to come off after a period of wear or the patient may have skin that is susceptible to itching or irritation. The wearing of ECG electrodes can aggravate such skin conditions. Thus, a patient may want or need to periodically remove or replace ECG electrodes during a long-term ECG monitoring period, whether to replace a dislodged electrode, reestablish better adhesion, alleviate itching or irritation, allow for cleansing of the skin, allow for showering and exercise, or for other purpose.

Following replacement, the monitor recorder 14 is again snapped into the electrode patch 15 (step 152) and monitoring resumes (step 153). The ability to transfer the same monitor recorder 14 to successive electrode patches 15 during a period of extended wear monitoring is advantageous not to just diagnose cardiac rhythm disorders and other physiological events of potential concern, but to do extremely long term monitoring, such as following up on cardiac surgery, ablation procedures, or medical device implantation. In these cases, several weeks of monitoring or more may be needed. In addition, some IMDs, such as pacemakers or implantable cardioverter defibrillators, incorporate a loop recorder that will capture cardiac events over a fixed time window. If the telemetry recorded by the IMD is not downloaded in time, cardiac events that occurred at a time preceding the fixed time window will be overwritten by the IMD and therefore lost. The monitor recorder 14 provides continuity of monitoring that acts to prevent loss of cardiac event data. In a further embodiment, the firmware executed by the microcontroller 61 of the monitor recorder 14 can be optimized for minimal power consumption and additional flash memory for storing monitoring data can be added to achieve a multi-week monitor recorder 14 that can be snapped into a fresh new electrode patch 15 every seven days, or other interval, for weeks or even months on end.

Upon the conclusion of monitoring, the monitor recorder 14 is removed (step 154) and recorded ECG and physiological telemetry are downloaded (step 155). For instance, a download station can be physically interfaced to the external connector 65 of the monitor recorder 14 to initiate and conduct downloading, as described *supra* with reference to FIGURE 15.

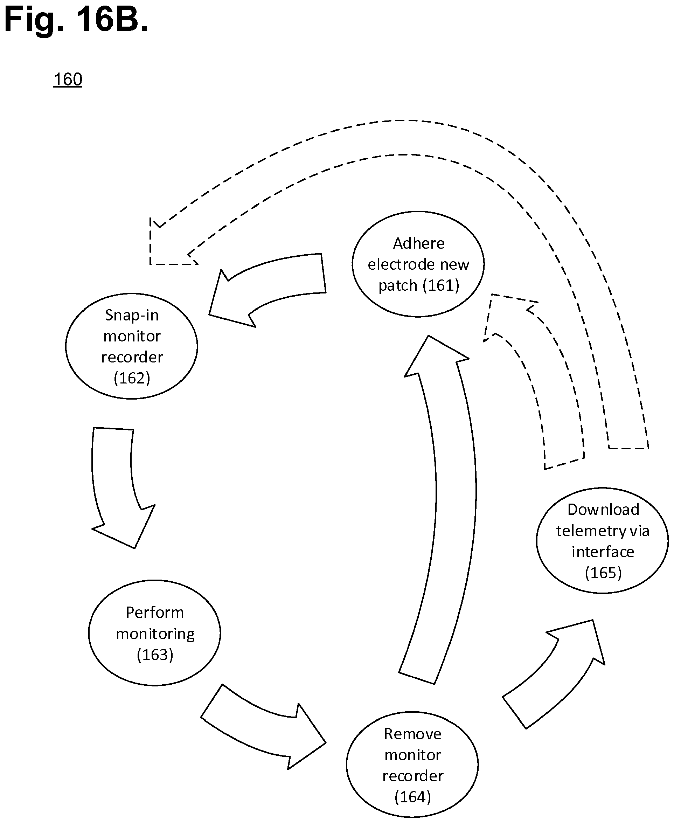

In a further embodiment, the monitoring period can be of indeterminate duration. Referring next to FIGURE 16B, a similar series of operations are followed with respect to replacement of electrode patches 15, reinsertion of the same monitor recorder 14, and eventual download of ECG and physiological telemetry (steps 161-165), as described *supra* with reference to FIGURE 16A. However, the flash memory 62 (shown in FIGURE 9) in the circuitry 60 of the monitor recorder 14 has a finite capacity. Following successful downloading of stored data, the flash memory 62 can be cleared to restore storage capacity and monitoring can resume once more, either by first adhering a new electrode patch 15 (step 161) or by snapping the monitor recorder 14 into an already-adhered electrode patch 15 (step 162). The foregoing expanded series of operations, to include reuse of the same monitor recorder 14 following data download, allows monitoring to continue indefinitely and without the kinds of interruptions that often affect conventional approaches, including the retrieval of monitoring data only by first making an appointment with a medical professional.

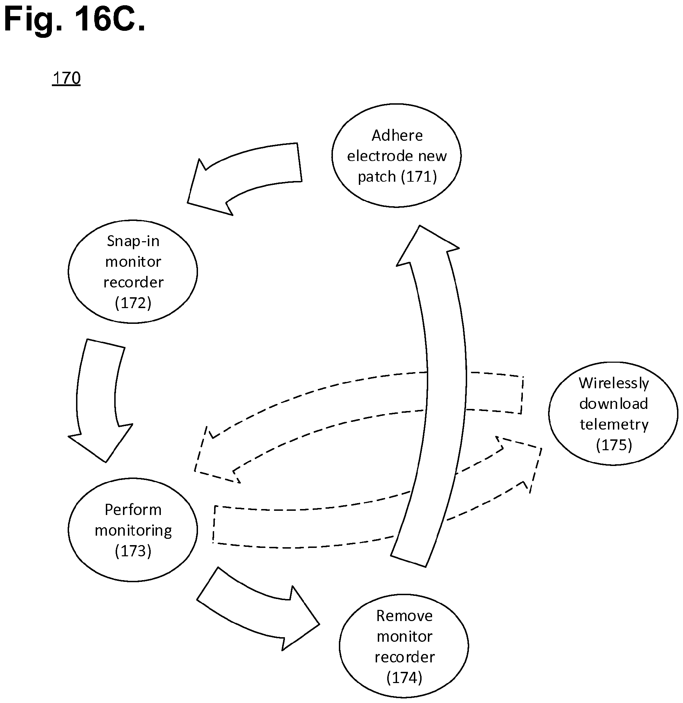

In a still further embodiment, when the monitor recorder 14 is equipped with a wireless transceiver, the use of a download station can be skipped. Referring last to FIGURE 16C, a similar series of operations are followed with respect to replacement of electrode patches 15 and reinsertion of the same monitor recorder 14 (steps 171-174), as described *supra* with reference to FIGURE 16A. However, recorded ECG and physiological telemetry are downloaded wirelessly (step 175), such as described in commonly-assigned U.S. Patent application, Serial No. 14/082,071, cited *supra*. The recorded ECG and physiological telemetry can even be downloaded wirelessly directly from a monitor recorder 14 during monitoring while still snapped into the non-conductive receptacle 25 on the electrode patch 15. The wireless interfacing enables monitoring to continue for an open-ended period of time, as the downloading of the recorded ECG and physiological telemetry will continually free up onboard storage space. Further, wireless interfacing simplifies patient use, as the patient (or caregiver) only need worry about placing (and replacing) electrode patches 15 and inserting the monitor recorder 14. Still other forms of practical use of the extended wear electrocardiography monitors 12 are possible.

The circuit trace and ECG electrodes components of the electrode patch 15 can be structurally simplified. In a still further embodiment, the flexible circuit 32 (shown in FIGURE 5) and distal ECG electrode 38 and proximal ECG electrode 39 (shown in FIGURE 6) are replaced with a pair of interlaced flexile wires. The interlacing of flexile wires through the flexible backing 20 reduces both manufacturing costs and environmental impact, as further described *infra*. The flexible circuit and ECG electrodes are replaced with a pair of flexile wires that serve as both electrode circuit traces and electrode signal pickups. FIGURE 17 is a perspective view 180 of an extended wear electrode patch 15 with a flexile wire electrode assembly in accordance with a still further embodiment. The flexible backing 20 maintains the unique narrow "hourglass"-like shape that aids long term extended wear, particularly in women, as described *supra* with reference to FIGURE 4. For clarity, the non-conductive receptacle 25 is omitted to show the exposed battery printed circuit board 182 that is adhered underneath the non-conductive receptacle 25 to the proximal end 31 of the flexible backing 20. Instead of employing flexible circuits, a pair of flexile wires are separately interlaced or sewn into the flexible backing 20 to serve as circuit connections for an anode electrode lead and for a cathode electrode lead.

To form a distal electrode assembly, a distal wire 181 is interlaced into the distal end 30 of the flexible backing 20, continues along an axial path through the narrow longitudinal midsection of the elongated strip, and electrically connects to the battery printed circuit board 182 on the proximal end 31 of the flexible backing 20. The distal wire 181 is connected to the battery printed circuit board 182 by stripping the distal wire 181 of insulation, if applicable, and interlacing or sewing the uninsulated end of the distal wire 181 directly into an exposed circuit trace 183. The distal wire-to-battery printed circuit board connection can be made, for instance, by back stitching the distal wire 181 back and forth across the edge of the battery printed circuit board 182. Similarly, to form a proximal electrode assembly, a proximal wire (not shown) is interlaced into the proximal end 31 of the flexible backing 20. The proximal wire is connected to the battery printed circuit board 182 by stripping the proximal wire of insulation, if applicable, and interlacing or sewing the uninsulated end of the proximal wire directly into an exposed circuit trace 184. The resulting flexile wire connections both establish electrical connections and help to affix the battery printed circuit board 182 to the flexible backing 20.

The battery printed circuit board 182 is provided with a battery compartment 36. A set of electrical pads 34 are formed on the battery printed circuit board 182. The electrical pads 34 electrically interface the battery printed circuit board 182 with a monitor recorder 14 when fitted into the non-conductive receptacle 25. The battery compartment 36 contains a spring 185 and a clasp 186, or similar assembly, to hold a battery (not shown) in place and electrically interfaces the battery to the electrical pads 34 through a pair battery leads 187 for powering the electrocardiography monitor 14. Other types of battery compartment are possible. The battery contained within the battery compartment 36 can be replaceable, rechargeable, or disposable.

In a yet further embodiment, the circuit board and non-conductive receptacle 25 are replaced by a combined housing that includes a battery compartment and a plurality of electrical pads. The housing can be affixed to the proximal end of the elongated strip through the interlacing or sewing of the flexile wires or other wires or threads.

The core of the flexile wires may be made from a solid, stranded, or braided conductive metal or metal compounds. In general, a solid wire will be less flexible than a stranded wire with the same total cross-sectional area, but will provide more mechanical rigidity than the stranded wire. The conductive core may be copper, aluminum, silver, or other material. The pair of the flexile wires may be provided as insulated wire. In one embodiment, the flexile wires are made from a magnet wire from Belden Cable, catalogue number 8051, with a solid core of AWG 22 with bare copper as conductor material and insulated by polyurethane or nylon. Still other types of flexile wires are possible. In a further embodiment, conductive ink or graphene can be used to print electrical connections, either in combination with or in place of the flexile wires.

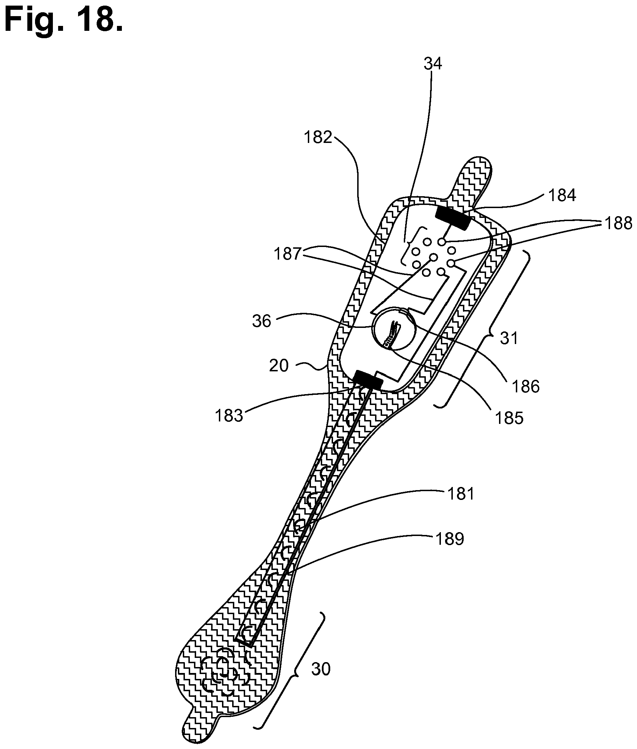

In a still further embodiment, the flexile wires are uninsulated. FIGURE 18 is perspective view of the flexile wire electrode assembly from FIGURE 17, with a layer of insulating material 189 shielding a bare uninsulated distal wire 181 around the midsection on the contact side of the flexible backing. On the contact side of the proximal and distal ends of the flexible backing, only the portions of the flexile wires serving as electrode signal pickups are electrically exposed and the rest of the flexile wire on the contact side outside of the proximal and distal ends are shielded from electrical contact. The bare uninsulated distal wire 181 may be insulated using a layer of plastic, rubber-like polymers, or varnish, or by an additional layer of gauze or adhesive (or non-adhesive) gel. The bare uninsulated wire 181 on the non-contact side of the flexible backing may be insulated or can simply be left uninsulated.

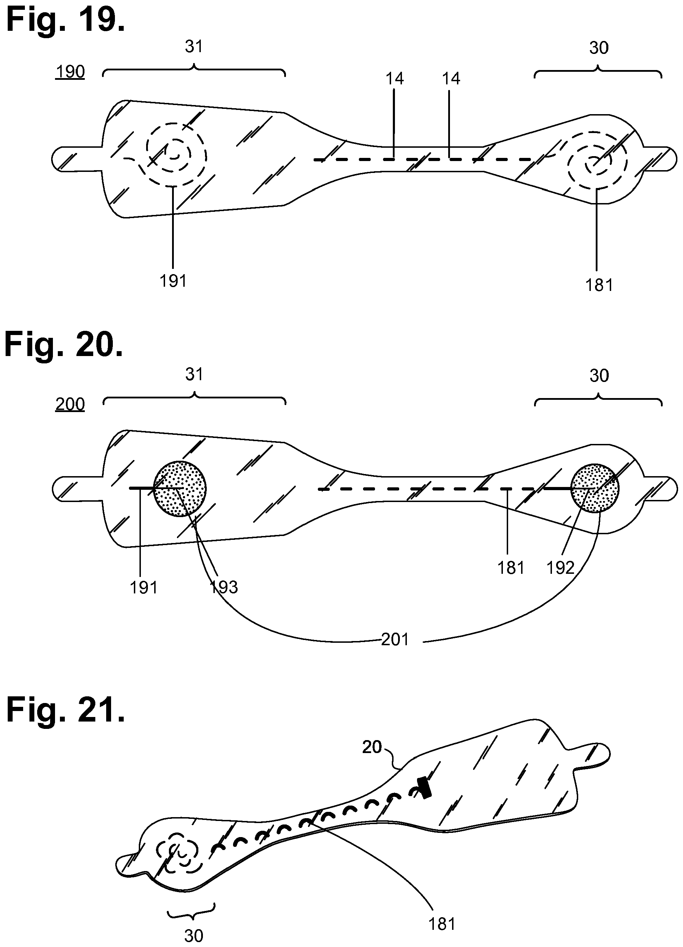

Both end portions of the pair of flexile wires are typically placed uninsulated on the contact surface of the flexible backing 20 to form a pair of electrode signal pickups. FIGURE 19 is a bottom view 190 of the flexile wire electrode assembly as shown in FIGURE 17. When adhered to the skin during use, the uninsulated end portions of the distal wire 181 and the proximal wire 191 enable the monitor recorder 14 to measure dermal electrical potential differentials. At the proximal and distal ends of the flexible backing 20, the uninsulated end portions of the flexile wires may be configured into an appropriate pattern to provide an electrode signal pickup, which would typically be a spiral shape formed by guiding the flexile wire along an inwardly spiraling pattern. The surface area of the electrode pickups can also be variable, such as by selectively removing some or all of the insulation on the contact surface. For example, an electrode signal pickup arranged by sewing insulated flexile wire in a spiral pattern could have a crescent-shaped cutout of uninsulated flexile wire facing towards the signal source.

In a still yet further embodiment, the flexile wires are left freely riding on the contact surfaces on the distal and proximal ends of the flexible backing, rather than being interlaced into the ends of the flexible backing 20. FIGURE 20 is a bottom view 200 of a flexile wire electrode assembly in accordance with a still yet further embodiment. The distal wire 181 is interlaced onto the midsection and extends an exposed end portion 192 onto the distal end 30. The proximal wire 191 extends an exposed end portion 193 onto the proximal end 31. The exposed end portions 192 and 193, not shielded with insulation, are further embedded within an electrically conductive adhesive 201. The adhesive 201 makes contact to skin during use and conducts skin electrical potentials to the monitor recorder 14 (not shown) via the flexile wires. The adhesive 201 can be formed from electrically conductive, non-irritating adhesive, such as hydrocolloid.

The distal wire 181 is interlaced or sewn through the longitudinal midsection of the flexible backing 20 and takes the place of the flexible circuit 32. FIGURE 21 is a perspective view showing the longitudinal midsection of the flexible backing of the electrode assembly from FIGURE 17. Various stitching patterns may be adopted to provide a proper combination of rigidity and flexibility. In simplest form, the distal wire 181 can be manually threaded through a plurality of holes provided at regularly-spaced intervals along an axial path defined between the battery printed circuit board 182 (not shown) and the distal end 30 of the flexible backing 20. The distal wire 181 can be threaded through the plurality of holes by stitching the flexile wire as a single "thread." Other types of stitching patterns or stitching of multiple "threads" could also be used, as well as using a sewing machine or similar device to machine-stitch the distal wire 181 into place, as further described *infra*. Further, the path of the distal wire 181 need not be limited to a straight line from the distal to the proximal end of the flexible backing 20.

While the invention has been particularly shown and described as referenced to the embodiments thereof, those skilled in the art will understand that the foregoing and other changes in form and detail may be made therein without departing from the scope.

## Claims

1. An ambulatory electrocardiography monitor (12) optimized for capturing low amplitude cardiac action potential propagation, comprising:
a disposable extended wear electrode patch (15) comprising:
a flexible backing (20) comprising stretchable material defined as an elongated strip (21) with a narrow longitudinal midsection (23), each end of the flexible backing (20) comprising an adhesive contact surface (201);
a pair of electrocardiographic electrodes (38, 39) comprised on the contact surface of each end of the flexible backing (20), each electrocardiographic electrode (38, 39) conductively exposed for dermal adhesion and adapted to be positioned axially along the midline (16) of the sternum (13) for capturing action potential propagation; and
a battery (71); and
an ambulatory electrocardiography monitor recorder (14) comprising electronic circuitry (60) which comprises:
a low power microcontroller (61) operable to execute over an extended period under modular micro program control as specified in firmware (132);
an electrocardiographic front end circuit (63) under the control of the microcontroller (61) adapted to sense cardiac electrical potential differentials through the electrocardiographic electrodes (38, 39), which are provided to the microcontroller (61) as electrocardiographic signals representative of amplitudes of the action potential propagation; and
non-volatile memory (62) electrically interfaced with the microcontroller (61) and operable to continuously store samples of the electrocardiographic signals throughout the extended period;
**characterized in that** the disposable extended wear electrode patch (15) further comprises:
a non-conductive receptacle (25) affixed to a non-contacting surface of the flexible backing (20);
a docking interface positioned on the non-conductive receptacle (25) and comprising electrical contact mating pads (34);
a battery compartment (36) formed on a bottom surface of the non-conductive receptacle (25);
the battery (71) arranged to be placed within the battery compartment (36) electrically interfaced to a first set of the electrical contact mating pads (34) of the docking interface; and
a pair of flexible circuit traces (33, 37) affixed at each end of the flexible backing (20) with each circuit trace (33, 37) connecting one of the electrocardiographic electrodes (38, 39) to one of the electrical contact mating pads (34) of a second set of electrical contact mating pads (34) of the docking interface, at least one of the circuit traces (33, 37) adapted to extend along the narrow longitudinal midsection (23);
wherein the non-conductive receptacle (25) is affixed to the non-contacting surface of one of the ends of the flexible backing (20) and the battery compartment (36) is positioned closer to the other end of the flexible backing (20) than the electrical contact mating pads (34); and **in that**
the ambulatory electrocardiography monitor recorder (14) further comprises:
a wearable housing (50) adapted to securely fit into the receptacle (25); and
an external connector (65) comprising a set of electrical contacts (56) protruding from a surface of the wearable housing (50) that fits within the non-conductive receptacle (25), the set of the contacts (56) electrically and mechanically contacting the first set of the electrical contact mating pads (34) of the docking interface and the second set of the electrical contact mating pads (34) of the docking interface;
the electronic circuitry (60) provided within the wearable housing (50) and removably connected via the external connector (65) to the electrocardiographic electrodes (38, 39) via the second set of the electrical contact mating pads of the docking interface, and to the battery via the first set of the electrical contact mating pads of the docking interface, wherein:
the low power microcontroller (61) is configured to draw power from the battery (71) via the external connector (65); and
the non-volatile memory (62) is configured to draw power from the battery (71) via the external connector (65).

2. An electrocardiography monitor (12) in accordance with Claim 1, the microcontroller (61) further comprising:
an analog-to-digital converter operable to convert the electrocardiographic signals into digital representations of the cardiac activation wave front amplitudes;
at least one low pass filter comprised in the firmware (132); and
at least one high pass filter comprised in the firmware (132),
wherein the cardiac activation wave front amplitudes are passed through the at least one low pass filter and the at least one high pass filter following conversion into the digital representations.

3. An electrocardiography monitor (12)
in accordance with Claim 2, the microcontroller (61) further comprising:
a compression algorithm comprised in the firmware (132),
wherein the cardiac activation wave front amplitudes are compressed with the compression algorithm into compressed digital representations prior to being stored in the non-volatile.

4. An electrocardiography monitor (12) in accordance with Claim 1, wherein the battery (71) comprises a direct current power cell.

5. An electrocardiography monitor (12) in accordance with Claim 1, further comprising:
a pair of strain relief cutouts (41) comprised in the narrow longitudinal midsection (23) serving as a strain relief (40) that each face towards the other strain relief cutout (41), each strain relief cutout (41) partially extending transversely from and continuing longitudinally to a side of a flexible circuit (32) different than the other strain relief cutout (41), one of the pair of circuit traces following a path formed on the flexible circuit (32) between the pair of strain relief cutouts (41).

6. An electrocardiography monitor (12) in accordance with Claim 1, further comprising:
an actigraphy sensor (64) comprised within the ambulatory electrocardiography monitor recorder (14) and operable to detect movement, the actigraphy sensor (64) electrically interfaced with the micro-controller (61); and
the non-volatile memory (62) further operable to store samples of the movement detected by the actigraphy sensor (64).

7. An electrocardiography monitor (12) in accordance with Claim 1, further comprising:
a transceiver provided with the wearable housing and operable to provide remote control over the micro-controller (61).

8. An electrocardiography monitor (12) according to Claim 1, further comprising:
an expansion port comprised in the micro-controller (61) and electrically coupled to at least one of the electrical contact mating pads (34);
a physiology sensor comprised within the disposable extended wear electrode patch (15) and operable to sense physiology and to draw power from the battery (71), the physiology sensor electrically interfaced to the expansion port; and
the non-volatile memory (62) further operable through the expansion port to store samples of the physiology sensed by the physiology sensor.

9. An electrocardiography monitor (12) according to Claim 8,
wherein the physiology sensor is selected from the group consisting of an SpO2 sensor, a blood pressure sensor, a temperature sensor, a respiratory rate sensor, a glucose sensor, an air flow sensor, and a volumetric pressure sensor.

10. An electrocardiography monitor (12) according to Claim 1, further comprising:
an expansion port comprised in the micro-controller (61);
a physiology sensor comprised within the ambulatory electrocardiography monitor recorder (14) and operable to sense physiology and to draw power from the battery (71), the physiology sensor electrically interfaced to the expansion port; and
the non-volatile memory (62) further operable through the expansion port to store samples of the physiology sensed by the physiology sensor.

11. An electrocardiography monitor (12) according to Claim 10, wherein the physiology sensor is selected from the group consisting of an SpO2 sensor, a blood pressure sensor, a temperature sensor, a respiratory rate sensor, a glucose sensor, an air flow sensor, and a volumetric pressure sensor.

12. An electrocardiography monitor (12) according to Claim 1, further comprising:
a power up sequence stored as part of the firmware (132), wherein the microcontroller (61) is operable to execute the power up sequence upon the power cell being electronically coupled to the electronic circuitry (60).

13. An electrocardiography monitor (12) according to Claim 12, the power up sequence comprising one or more of a battery voltage checking procedure, a flash memory state checking procedure, and a micro-controller diagnostic procedure.

14. An electrocardiography monitor (12) according to Claim 1, further comprising:
a buzzer (67) comprised in the electronic circuitry (60) configured to confirm an execution of the power up sequence.

15. An electrocardiography monitor (12) in accordance with Claim 1, further comprising:
a user-operable control (66) external to the wearable housing (50).

16. An electrocardiography monitor (12) according to claim 1, wherein the ambulatory electrocardiography monitor (14) removably snaps into the electrically non-conductive receptacle.

17. An electrocardiography monitor (12) according to claim 16,
wherein the non-conductive receptacle is provided with a retention catch (26) and tension clip (27) molded into the non-conductive receptacle (25) to conformably receive and securely hold the ambulatory electrocardiography monitor (14) in place.

## Patentansprüche

1. Ambulatorischer Bildschirm für Elektrokardiographie (12), der optimiert ist, um die Ausbreitung des Aktionspotentials des Herzens mit niedriger Amplitude zu erfassen, und der Folgendes umfasst:
ein Einwegelektrodenpflaster mit verlängerter Tragedauer (15), das Folgendes umfasst:
eine flexible Unterlage (20), die ein dehnbares Material umfasst, das als ein verlängerter Streifen (21) mit einem schmalen Längsmittelabschnitt (23) definiert ist, wobei jedes Ende der flexiblen Unterlage (20) eine Haftkontaktfläche (201) umfasst;
ein Paar von elektrokardiographischen Elektroden (38, 39), die auf der Kontaktfläche von jedem Ende der flexiblen Unterlage (20) umfasst sind, wobei jede elektrokardiographische Elektrode (38, 39) für dermale Haftung leitfähig freigelegt ist und geeignet ist, um axial entlang der Mittellinie (16) des Brustbeins (13) positioniert zu werden, um die Ausbreitung des Aktionspotentials zu erfassen; und
eine Batterie (71); und
einen ambulatorischen Bildschirmrekorder für Elektrokardiographie (14), der einen elektronischen Schaltkreis (60) umfasst, der Folgendes umfasst:
eine Mikrosteuerung (61) mit niedriger Leistung, die betreibbar ist, um über einen längeren Zeitraum unter einer modularen Mikroprogrammsteuerung, wie in der Firmware (132) angegeben, ausgeführt zu werden;
eine elektrokardiographische Eingangsschaltung (63), die von der Mikrosteuerung (61) gesteuert wird und geeignet ist, um elektrische Potentialdifferenzen des Herzens durch die elektrokardiographischen Elektroden (38, 39) zu erfassen, die der Mikrosteuerung (61) als elektrokardiographische Signale bereitgestellt werden, die die Amplituden der Ausbreitung des Aktionspotentials darstellen; und
einen nichtflüchtigen Speicher (62), der elektrisch an die Mikrosteuerung (61) angeschlossen ist und betreibbar ist, um kontinuierlich Proben der elektrokardiographischen Signale über einen längeren Zeitraum zu speichern;
**dadurch gekennzeichnet, dass** das Einwegelektrodenpflaster (15) mit verlängerter Tragedauer ferner Folgendes umfasst:
einen nicht-leitfähigen Aufnahmebehälter (25), der an einer kontaktlosen Oberfläche der flexiblen Unterlage (20) befestigt ist;
eine Andockschnittstelle, die auf dem nicht-leitfähigen Aufnahmebehälter (25) positioniert ist und elektrische Kontaktanpassungskissen (34) umfasst;
ein Batterieabteil (36), das auf einer Bodenoberfläche des nicht-leitfähigen Aufnahmebehälters (25) ausgebildet ist;
wobei die Batterie (71) angeordnet ist, um innerhalb des Batterieabteils (36) untergebracht zu werden, das elektrisch an einen ersten Satz der elektrischen Kontaktanpassungskissen (34) der Andockschnittstelle angeschlossen ist; und
ein Paar flexibler Schaltungsspuren (33, 37), die an jedem Ende der flexiblen Unterlage (20) befestigt sind, wobei jede Schaltungsspur (33, 37) eine der elektrokardiographischen Elektroden (38, 39) an eine der elektrischen Kontaktanpassungskissen (34) eines zweiten Satzes von elektrischen Kontaktanpassungskissen (34) der Andockschnittstelle verbindet, wobei mindestens eine der Schaltungsspuren (33, 37) geeignet ist, um sich entlang des schmalen Längsmittelabschnitts (23) zu erstrecken;
wobei der nicht-leitfähige Aufnahmebehälter (25) an der kontaktlosen Oberfläche eines der Enden der flexiblen Unterlage (20) befestigt ist und das Batterieabteil (36) näher an dem anderen Ende der flexiblen Unterlage (20) positioniert ist als die elektrischen Kontaktanpassungskissen (34); und dadurch, dass
der ambulatorische elektrokardiographische Bildschirmrekorder (14) ferner Folgendes umfasst:
ein tragbares Gehäuse (50), das geeignet ist, um sicher in den Aufnahmebehälter (25) zu passen; und
einen äußeren Verbinder (65), der einen Satz von elektrischen Kontakten (56) umfasst, die von einer Oberfläche des tragbaren Gehäuses (50) hervorstehen, das in den nicht-leitfähigen Aufnahmebehälter (25) passt, wobei der Satz der Kontakte (56) den ersten Satz der elektrischen Kontaktanpassungskissen (34) der Andockschnittstelle und den zweiten Satz der elektrischen Kontaktanpassungskissen (34) der Andockschnittstelle elektrisch und mechanisch kontaktiert;
den elektronischen Schaltkreis (60), der innerhalb des tragbaren Gehäuses (50) bereitgestellt ist und über den äußeren Verbinder (65) entfernbar mit den elektrokardiographischen Elektroden (38, 39) über den zweiten Satz der elektrischen Kontaktanpassungskissen der Andockschnittstelle und mit der Batterie über den ersten Satz der elektrischen Kontaktanpassungkissen der Andockschnittstelle verbunden ist, wobei:
die Mikrosteuerung (61) mit niedriger Leistung konfiguriert ist, um Leistung von der Batterie (71) über den äußeren Verbinder (65) zu ziehen; und
wobei der nichtflüchtige Speicher (62) konfiguriert ist, um Leistung von der Batterie (71) über den äußeren Verbinder (65) zu ziehen.

2. Bildschirm für Elektrokardiographie (12) nach Anspruch 1, wobei die Mikrosteuerung (61) ferner Folgendes umfasst:
einen Analog-Digital-Wandler, der betreibbar ist, um die elektrokardiographischen Signale in digitale Darstellungen der Wellenfrontamplituden der Herzaktivierung umzuwandeln;
mindestens einen Tiefpassfilter, der in der Firmware (132) umfasst ist; und
mindestens einen Hochpassfilter, der in der Firmware (132) umfasst ist, wobei die Wellenfrontamplituden der Herzaktivierung nach der Umwandlung in die digitalen Darstellungen durch den mindestens einen Tiefpassfilter und den mindestens einen Hochpassfilter geführt werden.

3. Bildschirm für Elektrokardiographie (12) nach Anspruch 2, wobei die Mikrosteuerung (61) ferner Folgendes umfasst:
einen Kompressionsalgorithmus, der in der Firmware (132) umfasst ist, wobei die Wellenfrontamplituden der Herzaktivierung mit dem Kompressionsalgorithmus zu komprimierten digitalen Darstellungen komprimiert werden, bevor sie in dem nichtflüchtigen Speicher gespeichert werden.

4. Bildschirm für Elektrokardiographie (12) nach Anspruch 1, wobei die Batterie (71) eine Gleichstromleistungszelle umfasst.

5. Bildschirm für Elektrokardiographie (12) nach Anspruch 1, der ferner Folgendes umfasst:
ein Paar von Zugentlastungsausschnitten (41), die in dem schmalen Längsmittelabschnitt (23) umfasst sind, die als Zugentlastung (40) dienen, die jeweils dem anderen Zugentlastungsausschnitt (41) zugewandt sind, wobei sich jeder Zugentlastungsausschnitt (41) teilweise von einer Seite einer flexiblen Schaltung (32), die sich von dem anderen Zugentlastungsausschnitt (41) unterscheidet, quer erstreckt und sich längs zu derselben fortsetzt, wobei eine des Paars von Schaltungspuren einem Pfad folgt, der auf der flexiblen Schaltung (32) zwischen dem Paar von Zugentlastungsausschnitten (41) gebildet ist.

6. Bildschirm für Elektrokardiographie (12) nach Anspruch 1, der ferner Folgendes umfasst:
einen Aktigraphiesensor (64), der innerhalb des ambulatorischen Bildschirmrekorders für Elektrokardiographie (14) umfasst ist und betreibbar ist, um eine Bewegung zu erkennen, wobei der Aktigraphiesensor (64) elektrisch an die Mikrosteuerung (61) angeschlossen ist; und
wobei der nichtflüchtige Speicher (62) ferner betreibbar ist, um Proben der Bewegung, die von dem Aktigraphiesensor (64) erkannt wird, zu speichern.

7. Bildschirm für Elektrokardiographie (12) nach Anspruch 1, der ferner Folgendes umfasst:
einen Sendeempfänger, der mit dem tragbaren Gehäuse bereitgestellt wird und betreibbar ist, um eine Fernsteuerung über der Mikrosteuerung (61) bereitzustellen.

8. Bildschirm für Elektrokardiographie (12) nach Anspruch 1, der ferner Folgendes umfasst:
einen Erweiterungsanschluss, der in der Mikrosteuerung (61) umfasst und elektrisch an mindestens einem der elektrischen Kontaktanpassungskissen (34) gekoppelt ist;
einen Physiologiesensor, der in dem Einwegelektrodenpflaster mit verlängerter Tragedauer (15) umfasst ist und betreibbar ist, um die Physiologie zu erfassen und Leistung von der Batterie (71) zu ziehen, wobei der Physiologiesensor elektrisch an den Erweiterungsanschluss angeschlossen ist; und
den nichtflüchtigen Speicher (62), der ferner durch den Erweiterungsanschluss betreibbar ist, um Proben der vom Physiologiesensor erfassten Physiologie zu speichern.

9. Bildschirm für Elektrokardiographie (12) nach Anspruch 8, wobei der Physiologiesensor aus der Gruppe ausgewählt ist, die aus einem SpO2-Sensor, einem Blutdrucksensor, einem Temperatursensor, einem Atemfrequenzsensor, einem Glukosesensor, einem Luftstromsensor und einem volumetrischen Drucksensor besteht.

10. Bildschirm für Elektrokardiographie (12) nach Anspruch 1, der ferner Folgendes umfasst:
einen Erweiterungsanschluss, der in der Mikrosteuerung (61) umfasst ist;
einen Physiologiesensor, der innerhalb des ambulatorischen Bildschirmrekorders für Elektrokardiographie (14) umfasst ist und betreibbar ist, um die Physiologie zu erfassen und Leistung von der Batterie (71) zu ziehen, wobei der Physiologiesensor elektrisch an den Erweiterungsanschluss angeschlossen ist; und
den nichtflüchtigen Speicher (62), der ferner durch den Erweiterungsanschluss betreibbar ist, um Proben der vom Physiologiesensor erfassten Physiologie zu speichern.

11. Bildschirm für Elektrokardiographie (12) nach Anspruch 10, wobei der Physiologiesensor aus der Gruppe ausgewählt ist, die aus einem SpO2-Sensor, einem Blutdrucksensor, einem Temperatursensor, einem Atemfrequenzsensor, einem Glukosesensor, einem Luftstromsensor und einem volumetrischen Drucksensor besteht.

12. Bildschirm für Elektrokardiographie (12) nach Anspruch 1, der ferner Folgendes umfasst:
eine Einschaltsequenz, die als Teil der Firmware (132) gespeichert ist, wobei die Mikrosteuerung (61) betreibbar ist, um die Einschaltsequenz auszuführen, nachdem die Leistungszelle elektronisch an den elektronischen Schaltkreis (60) gekoppelt wurde.

13. Bildschirm für Elektrokardiographie (12) nach Anspruch 12, wobei die Einschaltsequenz ein oder mehrere Methoden zum Überprüfen der Batteriespannung, eine Methode zum Überprüfen des Zustands des Flash-Speichers und eine Diagnosemethode für die Mikrosteuerung umfasst.

14. Bildschirm für Elektrokardiographie (12) nach Anspruch 1, der ferner Folgendes umfasst:
einen Summer (67), der in dem elektronischen Schaltkreis (60) umfasst ist, der konfiguriert ist, um eine Ausführung der Einschaltsequenz zu bestätigen.

15. Bildschirm für Elektrokardiographie (12) nach Anspruch 1, der ferner Folgendes umfasst:
eine benutzerbedienbare Steuerung (66) außerhalb des tragbaren Gehäuses (50).

16. Bildschirm für Elektrokardiographie (12) nach Anspruch 1, wobei der ambulatorische Bildschirm für Elektrokardiographie (14) entfernbar in den elektrisch nicht-leitfähigen Aufnahmebehälter einschnappt.

17. Bildschirm für Elektrokardiographie (12) nach Anspruch 16, wobei der nicht-leitfähige Aufnahmebehälter mit einem Haltehaken (26) und einer Spannklammer (27) bereitgestellt wird, die in den nicht-leitfähigen Aufnahmebehälter (25) eingegossen ist, um den ambulatorischen Bildschirm für Elektrokardiographie (14) konform zu empfangen und sicher an Ort und Stelle zu halten.

## Revendications

1. Moniteur d'électrocardiographie ambulatoire (12) optimisé pour capter la propagation de potentiels d'action cardiaques de faible amplitude, comprenant :
un timbre-électrode (15) jetable à port prolongé comprenant :
un support souple (20) comprenant un matériau extensible défini comme une bande allongée (21) comportant une section médiane longitudinale étroite (23), chaque extrémité du support souple (20) comprenant une surface de contact adhésive (201) ;
une paire d'électrodes électrocardiographiques (38, 39) comprises sur la surface de contact de chaque extrémité du support souple (20), chaque électrode électrocardiographique (38, 39) étant exposée de manière conductrice pour l'adhésion cutanée et apte à être positionnée axialement le long de la ligne médiane (16) du sternum (13) pour capter la propagation de potentiels d'action ; et
une batterie (71) ; et
un moniteur enregistreur d'électrocardiographie ambulatoire (14) comprenant un ensemble de circuits électronique (60) qui comprend :
un microcontrôleur de faible puissance (61) pouvant être actionné pour s'exécuter pendant une période prolongée sous un contrôle de microprogramme modulaire comme spécifié dans le micrologiciel (132) ;
un circuit frontal électrocardiographique (63) sous le contrôle du microcontrôleur (61) apte à détecter les différentiels de potentiels électriques cardiaques à travers les électrodes électrocardiographiques (38, 39), qui sont fournies au microcontrôleur (61) sous forme de signaux électrocardiographiques représentant des amplitudes de la propagation de potentiels d'action ; et
une mémoire non volatile (62) interfacée électriquement par le microcontrôleur (61) et pouvant être actionnée pour stocker en continu des échantillons des signaux électrocardiographiques tout au long de la période prolongée ;
**caractérisé en ce que** le timbre-électrode (15) jetable à port prolongé comprend en outre :
un réceptacle non conducteur (25) fixé à une surface sans contact du support souple (20) ;
une interface d'accueil positionnée sur le réceptacle non conducteur (25) et comprenant des plots d'accouplement de contact électrique (34) ;
un compartiment de batterie (36) réalisé sur une surface inférieure du réceptacle non conducteur (25) ;
la batterie (71) agencée pour être placée à l'intérieur du compartiment de batterie (36) électriquement interfacée à un premier ensemble de plots d'accouplement de contact électrique (34) de l'interface d'accueil ; et
une paire de traces de circuit souples (33, 37) fixées à chaque extrémité du support souple (20), chaque trace de circuit (33,37) reliant l'une des électrodes électrocardiographiques (38, 39) à l'un des plots d'accouplement de contact électrique (34) d'un second ensemble de plots d'accouplement de contact électrique (34) de l'interface d'accueil, au moins l'une des traces de circuit (33,37) étant apte à s'étendre le long de la section médiane longitudinale étroite (23) ;
dans lequel le réceptacle non conducteur (25) est fixé à la surface sans contact de l'une des extrémités du support souple (20) et le compartiment de batterie (36) est positionné plus près de l'autre extrémité du support souple (20) que les plots d'accouplement de contact électrique (34) ; et en ce
le moniteur enregistreur d'électrocardiographie ambulatoire (14) comprend en outre :
un boîtier portable (50) apte à s'adapter solidement dans le réceptacle (25) ; et
un connecteur externe (65) comprenant un ensemble de contacts électriques (56) faisant saillie d'une surface du boîtier portable (50) qui s'adapte à l'intérieur du réceptacle non conducteur (25), l'ensemble des contacts (56) étant en contact électrique et mécanique avec le premier ensemble des plots d'accouplement de contact électrique (34) de l'interface d'accueil et le second ensemble des plots d'accouplement de contact électrique (34) de l'interface d'accueil ;
l'ensemble de circuits électroniques (60) prévus à l'intérieur du boîtier portable (50) et connectés de manière amovible par l'intermédiaire du connecteur externe (65) aux électrodes électrocardiographiques (38, 39) par l'intermédiaire du second ensemble de plots d'accouplement de contact électrique de l'interface d'accueil, et à la batterie par l'intermédiaire du premier ensemble de plots d'accouplement de contact électrique de l'interface d'accueil, dans lequel :
le microcontrôleur de faible puissance (61) est configuré pour tirer de l'énergie de la batterie (71) par l'intermédiaire du connecteur externe (65) ; et
la mémoire non volatile (62) est configurée pour tirer de l'énergie de la batterie (71) par l'intermédiaire du connecteur externe (65).

2. Moniteur d'électrocardiographie (12) selon la revendication 1, le microcontrôleur (61) comprenant en outre :
un convertisseur analogique-numérique pouvant être actionné pour convertir les signaux électrocardiographiques en représentations numériques des amplitudes de front d'onde d'activation cardiaque ;
au moins un filtre passe-bas compris dans le micrologiciel (132) ; et
au moins un filtre passe-haut compris dans le micrologiciel (132), dans lequel les amplitudes de front d'onde d'activation cardiaque sont passées à travers ledit au moins un filtre passe-bas et ledit au moins un filtre passe-haut après conversion en représentations numériques.

3. Moniteur d'électrocardiographie (12) selon la revendication 2, le microcontrôleur (61) comprenant en outre :
un algorithme de compression compris dans le micrologiciel (132), dans lequel les amplitudes de front d'onde d'activation cardiaque sont compressées par l'algorithme de compression en représentations numériques compressées avant d'être stockées dans la mémoire non volatile.

4. Moniteur d'électrocardiographie (12) selon la revendication 1, dans lequel la batterie (71) comprend une cellule de puissance à courant continu.

5. Moniteur d'électrocardiographie (12) selon la revendication 1, comprenant en outre :
une paire de découpes de décharge de traction (41) comprises dans la section médiane longitudinale étroite (23) servant de décharge de traction (40) qui sont orientés chacun vers l'autre découpe de décharge de traction (41), chaque découpe de décharge de traction (41) s'étendant de manière partiellement transversale à partir d'un côté d'un circuit souple (32) différent de l'autre coupe-circuit de décharge de traction (41) et continuant de manière longitudinale vers celui-ci, une trace de la paire de traces de circuit suivant un chemin formé sur le circuit souple (32) entre la paire de découpes de décharge de traction (41).

6. Moniteur d'électrocardiographie (12) selon la revendication 1, comprenant en outre :
un capteur d'actigraphie (64) compris dans le moniteur enregistreur d'électrocardiographie ambulatoire (14) et pouvant être actionné pour détecter un mouvement, le capteur d'actigraphie (64) étant électriquement interfacé par le microcontrôleur (61) ; et
la mémoire non volatile (62) pouvant en outre être actionnée pour stocker des échantillons du mouvement détecté par le capteur d'actigraphie (64).

7. Moniteur d'électrocardiographie (12) selon la revendication 1, comprenant en outre :
un émetteur-récepteur pourvu du boîtier portable et pouvant être actionné pour permettre une commande à distance du microcontrôleur (61).

8. Moniteur d'électrocardiographie (12) selon la revendication 1, comprenant en outre :
un port d'expansion compris dans le microcontrôleur (61) et couplé électriquement à au moins l'un des plots d'accouplement de contact électrique (34) ;
un capteur de physiologie compris à l'intérieur du timbre-électrode (15) jetable à port prolongé et pouvant être actionné pour détecter la physiologie et pour tirer de l'énergie de la batterie (71), le capteur de physiologie étant électriquement interfacé au port d'expansion ; et
la mémoire non volatile (62) pouvant en outre être actionnée par l'intermédiaire du port d'extension pour stocker des échantillons de la physiologie détectée par le capteur de physiologie.

9. Moniteur d'électrocardiographie (12) selon la revendication 8, dans lequel le capteur de physiologie est sélectionné dans le groupe constitué par un capteur SpO2, un capteur de pression artérielle, un capteur de température, un capteur de fréquence respiratoire, un capteur de glucose, un capteur d'écoulement d'air et un capteur de pression volumétrique.

10. Moniteur d'électrocardiographie (12) selon la revendication 1, comprenant en outre :
un port d'extension compris dans le microcontrôleur (61) ;
un capteur de physiologie compris à l'intérieur du moniteur enregistreur d'électrocardiographie ambulatoire (14) et pouvant être actionné pour détecter la physiologie et pour tirer l'énergie de la batterie (71), le capteur de physiologie étant électriquement interfacé au port d'expansion ; et
la mémoire non volatile (62) pouvant en outre être actionnée par l'intermédiaire du port d'extension pour stocker des échantillons de la physiologie détectée par le capteur de physiologie.

11. Moniteur d'électrocardiographie (12) selon la revendication 10, dans lequel le capteur de physiologie est sélectionné dans le groupe constitué par un capteur SpO2, un capteur de pression artérielle, un capteur de température, un capteur de fréquence respiratoire, un capteur de glucose, un capteur d'écoulement d'air et un capteur de pression volumétrique.

12. Moniteur d'électrocardiographie (12) selon la revendication 1, comprenant en outre :
une séquence de mise sous tension stockée en tant que partie du micrologiciel (132), dans lequel le microcontrôleur (61) peut être actionné pour exécuter la séquence de mise sous tension lorsque la cellule de puissance est couplée électroniquement à l'ensemble de circuits électroniques (60).

13. Moniteur d'électrocardiographie (12) selon la revendication 12, la séquence de mise sous tension comprenant une procédure de vérification de tension de batterie et/ou une procédure de vérification d'état de mémoire flash et/ou une procédure de diagnostic de microcontrôleur.

14. Moniteur d'électrocardiographie (12) selon la revendication 1, comprenant en outre :
une sonnerie (67) comprise dans l'ensemble de circuits électroniques (60) configurée pour confirmer une exécution de la séquence de mise sous tension.

15. Moniteur d'électrocardiographie (12) selon la revendication 1, comprenant en outre :
une commande pouvant être actionnée par l'utilisateur (66) externe au boîtier portable (50).

16. Moniteur d'électrocardiographie (12) selon la revendication 1, dans lequel le moniteur d'électrocardiographie ambulatoire (14) s'encliquète de manière amovible dans le réceptacle électriquement non conducteur.

17. Moniteur d'électrocardiographie (12) selon la revendication 16, dans lequel le réceptacle non conducteur est pourvu d'un loquet de rétention (26) et d'une pince de tension (27) moulés dans le réceptacle non conducteur (25) pour recevoir de manière conforme et maintenir solidement le moniteur d'électrocardiographie ambulatoire (14) en place.
